# FASCICULE DE BREVET EUROPEEN

(11) **EP 2 858 654 B1**
(45) Date de publication et mention de la délivrance du brevet: **21.09.2016**
(21) Numéro de dépôt: 13727233.2
(22) Date de dépôt: 07.06.2013
(51) Int. Cl.: A61K 31/191, A61K 31/192, A61K 31/194, A61K 31/353, C12P 7/40, C12P 7/42, C12P 7/46, C12P 7/48, C12P 17/06, A61K 36/45

(54) **EXTRAIT DE CANNEBERGE UTILE DANS LE TRAITEMENT ET LA PRÉVENTION DES INFECTIONS URINAIRES**
PREISELBEEREXTRAKT ZUR BEHANDLUNG UND VORBEUGUNG VON HARNWEGSINFEKTIONEN
CRANBERRY EXTRACT FOR TREATING AND PREVENTING URINARY TRACT INFECTIONS

(30) Priorité: 07.06.2012 FR 1255324
(43) Date de publication de la demande: 15.04.2015
(73) Titulaire: Diana Naturals, 35560 Antrain (FR)
(72) Inventeur: SANONER, Philippe, F-50160 Torigny Sur Vire (FR); BOCHARD, Valérie, F-35420 Le Ferre (FR); CHARISSOU, Lucie, F-35300 Fougeres (FR); LASTIQUE, Bénédicte, F-35630 Hede (FR); JACOB, Morgane, F-35340 Liffre (FR); THOMAS, Patrice, F-35250 Saint Medard sur Ille (FR)
(74) Mandataire: Regimbeau
(86) Numéro de dépôt international: PCT/EP2013/061852
(87) Numéro de publication internationale: WO 2013/182702

(56) Documents cités:
- EP-A1- 2 033 641
- US-A1- 2010 028 468

## Description

### DOMAINE DE L'INVENTION

La présente invention concerne un extrait concentré de canneberge *(Vaccinium macrocarpon*) dont la composition complexe permet d'augmenter ses effets antibactériens, utile pour la prévention ou le traitement des infections urinaires, et notamment pour le traitement préventif ou anti-récidive d'infections urinaires. L'invention concerne également un procédé de préparation d'un tel extrait, des compositions alimentaires, nutraceutiques ou pharmaceutiques comprenant l'extrait et son utilisation dans le traitement ou la prévention des infections urinaires.

### ART ANTERIEUR

Les infections urinaires (UTIs) sont parmi les maladies infectieuses les plus répandues, et constituent un budget de santé considérable pour la société. Les micro-organismes peuvent atteindre les voies urinaires par voie hématogène ou lymphatique, mais la plupart des preuves cliniques et expérimentales montrent que l'ascension de l'urètre par les micro-organismes constitue la voie la plus commune qui conduit à une infection urinaire, en particulier par les organismes d'origine entérique (*Escherichia coli* et autres Entérobactéries). Ceci fournit une explication logique à la plus grande fréquence des infections urinaires chez les femmes que chez les hommes et au risque accru d'infection à la suite de cathétérisme de la vessie ou de l'instrumentation.

Se basant sur l'utilisation traditionnelle de canneberge *(Vaccinium macrocarpon,* appelée également « canneberge d'Amérique » ou « grande airelle rouge d'Amérique », ou encore « atoca » ou « ataca » au Québec, en anglais « cranberry » ou « American cranberry »), de nombreux ingrédients issus de fruit ou de jus sont utilisés sur le marché des compléments alimentaires en tant qu'agent curatif ou préventif permettant de limiter le risque d'apparition d'infections urinaires. Des liens épidémiologiques et cliniques entre la consommation de canneberge d'Amérique et l'apparition d'infections urinaires ont été mis en évidence.

La canneberge est un arbuste de petite taille (pas plus de 30 cm) qui croît spontanément uniquement dans l'Est de l'Amérique du Nord depuis les Carolines jusqu'au Canada. Sa présence caractérise les tourbières acides. Le fruit est une petite baie mesurant 10 à 20 mm de diamètre. A maturité, sa coloration est rouge vif et sa saveur acidulée et astringente.

La baie de canneberge se caractérise par sa richesse en flavonoïdes et comprend de nombreux types de composés différents, parmi lesquels :
- Des acides organiques, et notamment de l'acide quinique, l'acide citrique, l'acide malique et les acides phénoliques tels que les acides benzoïques, hydroxy-benzoïques et hydroxy-cinnamiques,
- Des anthocyanes (également appelés « anthocyanosides »),
- Des flavanols, et notamment :
   ∘ Des flavan-3-ol monomères tels que la catéchine, l'épicatéchine, la gallocatéchine, et l'épigallocatéchine,
   ∘ Des flavan-3-ols polymères, les proanthocyanidols (également appelés « proanthocyanidines » (anglais) ou « tannins condensés »),
- Des gallotanins et ellagitanins (également appelés tanins hydrolysables),
- Des flavonols, comme la quercétine par exemple, sous forme glycosylée et/ou aglycone.

La canneberge comprend différents acides organiques, tels que l'acide quinique, l'acide citrique ou l'acide malique. Pendant longtemps, l'effet de la consommation de jus de canneberge a été considéré comme lié à une acidification des urines permettant de limiter la croissance des bactéries uropathogéniques en diminuant le pH urinaire.

Ainsi, US2010/028468 décrit une composition pour traiter les infections urinaires, comprenant une formulation de Thymoquinone et des fruits, jus ou extraits de canneberge. Un extrait de canneberge susceptible d'être utilisé est un extrait à 5:1 standardisé pour avoir une teneur en acide quinique de 3,4% (le reste de sa composition n'est pas précisé). Cependant, il est rappelé dans l'introduction que l'efficacité thérapeutique des extraits de canneberge pour le traitement des infections urinaires n'a pas été démontrée.

En effet, le mécanisme d'action lié à une acidification des urines est trop simplificateur, ces acides organiques étant en réalité métabolisés in vivo et n'atteignant donc pas intacts les voies urinaires. Ainsi, bien que les cellules humaines ne métabolisent pas l'acide quinique, le matériel enzymatique de la flore intestinale permet de réduire l'acide quinique en acide benzoïque au niveau du colon.

Parmi les acides organiques, la canneberge est particulièrement concentrée en différents acides phénoliques dont des acides benzoïques, hydroxy-benzoïques et hydroxy-cinnamiques. Elle contient ainsi 0,05g/100g d'acide hydroxy-benzoïques (essentiellement représentés par de l'acide benzoïque), et moins de 0,1g/100g d'acides hydroxycinnamiques représentés essentiellement par les acides p-coumarique, sinapique et caféique. Elle comprend également d'autres acides organiques que les acides phénoliques, et notamment de l'acide quinique, qui est un intermédiaire de la synthèse du noyau benzénique via la voie shikimate (4-hydroxybenzoique), qui permet la synthèse des acides aminés aromatiques, des phenyl-propanoïdes et composés phénoliques acides hydroxycinnamiques et flavonoïdes.

Les acides phénoliques, et plus particulièrement les plus apolaires d'entre eux (tel que l'acide benzoïque) perturbent les possibilités d'échanges métaboliques des transporteurs des membranes bactériennes et ont donc un effet bactéricide.

Cependant, les acides phénoliques sont bio-disponibles et facilement transportés à travers la barrière intestinale de l'intestin grêle via les transporteurs des carboxylates, (Cong et al., 2001) et, une fois dans la circulation entéro-hépatique, sont ensuite conjugués avec de la glycine pour former de l'acide hippurique, pour être enfin excrétés dans l'urine par les reins. L'activité bactéricide de l'acide hippurique est moins importante que celle des acides phénoliques. L'activité antibactérienne directement apportées par les acides phénoliques de la canneberge est donc dans ce cas potentiellement diminuée par leur biodisponibilité dans l'intestin grêle.

Par conséquent, les effets des acides organiques tels que l'acide quinique et les acides phénoliques sur la prévention ou la diminution de la survenue d'infections urinaires ne sont pas complètement clairs.

Depuis quelques années, l'intérêt pour la canneberge dans la prévention des infections urinaires s'est focalisé sur les composés de type proanthocyanidols (également appelés « proanthocyanidines » ou « tannins condensés », abrégés en « PAC » dans la présente description).

Les PACs sont des polymères d'unités flavanols (telles que la catéchine, l'épicatéchine, la gallocatéchine, et l'épigallocatéchine), qui peuvent être liées entre elles de différentes façons.

Notamment, deux types de liaisons ont été décrites :
- Des liaisons de type A, dans lesquelles les deux unités flavanol sont liées par deux liaisons covalentes, l'une entre le carbone C-4 de la première unité flavanol et le carbone C-8 de la deuxième unité flavanol (C-4 → C-8), l'autre étant une liaison éther entre le carbone C-2 de la première unité flavanol et le carbone C-7 de la deuxième unité flavanol (C-2 → O → C-7). La présence de PACs avec ce type de liaison est l'une des caractéristiques de la canneberge, peu de végétaux présentant des PACs avec ce type de liaison.
- Des liaisons de type B, dans lesquelles les deux unités flavanol sont liées par une seule liaison covalente, entre le carbone C-4 de la première unité flavanol et le carbone C-6 ou C-8 de la deuxième unité flavanol (liaison C-4 → C-6 ou C-4 → C-8). Ces liaisons sont également présentes dans les PACs de canneberge.

Les PACs de canneberge comportent à la fois des liaisons de type A et de type B et sont particulièrement polymérisés, avec un degré de polymérisation moyen de 15 unités dans le fruit (Gu et al., 2002), soit 80% des proanthocyanidols avec un degré de polymérisation supérieur à 5 et comportant 46% de liaisons interflavaniques de type A..

L'intérêt pour les PACs de canneberge provient du fait qu'il a été montré in vitro que ces composés sont capables, comme la plupart des tannins, d'interagir avec les parois des cellules du tractus urinaire et/ou avec les composants extracellulaires des bactéries limitant leurs possibles interactions, et donc potentiellement la fixation ou l'adhésion des bactéries sur les parois des voies urinaires. Cet effet tannant est donc supposé expliquer avoir un effet sur l'apparition et la persistance des bactéries uropathogéniques colonisant le tractus urinaire.

Ainsi, le brevet EP0752871B1 décrit des extraits d'une plante du genre Vaccinium (notamment de canneberge) enrichis en une fraction ayant des effets anti-adhésion sur les bactéries et dépourvu en sucres simples ou dimériques, en acides et en anthocyanes, purifié par chromatographie, en particulier à l'aide d'une colonne lipophile. La fraction ayant des effets anti-adhésion sur les bactéries correspond majoritairement aux PACs.

De manière similaire, la demande WO96/30033 décrit des PACs particuliers ayant un degré de polymérisation entre 2 et 18 et leur utilisation pour la prévention ou le traitement des infections urinaires via un effet anti-adhésion des bactéries. Ces PACs sont susceptibles d'être purifiés à partir de canneberge (voir Exemple I), par un procédé comprenant l'alcalinisation de la canneberge à un pH supérieur à 10 pour ioniser les groupes phénol des composés polyphénoliques en groupes phénoxydes, la précipitation au méthanol, la ré-acidification du produit obtenu pour reconvertir les groupes phénoxydes en phénols, et sa purification sur une colonne lipophile. L'extrait obtenu comprend des PACs et des flavonoïdes. Au vu du procédé de préparation (utilisation d'une colonne lipophile notamment), cet extrait ne comprend pas de quantités significatives d'acides organiques, tels que l'acide quinique ou les acides phénoliques (mais seulement éventuellement des quantités faibles résiduelles, largement inférieures à 5% en poids par rapport au poids total de l'extrait sec).

Le brevet EP1014969B1 décrit un extrait de PACs de plantes (notamment de canneberge) ayant un effet anti-adhésion de bactéries particulières sur des surfaces. Le dit extrait contenant un ou plusieurs proanthocyanidols avec 5 à 6 unités, dont au moins deux sont liées par une liaison de type A. Cet extrait peut être obtenu par un procédé comprenant une extraction par un solvant aqueux, une étape de purification par chromatographie avec une colonne lipophile ou par extraction avec un solvant non polaire, et une seconde étape de purification par chromatographie avec une colonne hydrophile-lipophile. L'extrait obtenu comprend des PACS, mais est dépourvu notamment en sucres, en acides organiques, en anthocyanes, et en flavonols.

La demande EP2108268A1 décrit la préparation d'un extrait de canneberge fortement enrichi en PACs de canneberge de degré de polymérisation supérieur à 5 et son utilisation pour diminuer la fixation de certaines bactéries E. *Coli* sur les parois des voies urinaires. L'extrait est obtenu par un procédé comprenant une extraction par un solvant organique (notamment un alcool) des fractions insolubles obtenues à partir de baies de canneberge. Il comprend au moins 15% en poids de PACs exprimées en équivalent procyanidine C1 par rapport au poids sec de l'extrait, une quantité d'anthocyanes similaire à celle de la baie de canneberge, des flavonols, et des résidus de sucres, de protéines, de fibres et de matières minérales. Etant obtenu à partir des fractions insolubles de canneberge, il ne comprend en revanche pas de quantités significatives d'acides organiques, tels que l'acide quinique ou les acides phénoliques (mais seulement éventuellement des quantités faibles résiduelles, largement inférieures à 5% en poids par rapport au poids total de l'extrait sec).

La demande WO2010/121203 décrit un extrait de canneberge destiné à réduire la contamination bactérienne dans des boissons, comprenant des PACs et des anthocyanes, mais comprenant des quantités résiduelles très limitées en sucres et acides organiques du fait d'une étape de purification sur une résine fixant les composés phénolique mais ne se liant pas de façon substantielle aux sucres et aux acides organiques. La description générale indique que la quantité résiduelle de sucres ou d'acides organiques est dans tous les cas inférieure à 5%, et les exemples d'extrait caractérisés dans la partie expérimentale comprennent tous moins de 1% de sucres et d'acides organiques résiduels.

Des effets bactéricides et bactériostatiques des PACs de canneberge ont également été mis en évidence in vitro (Sanchez-Patan et al., 2012).

EP2033641A1 décrit un procédé de préparation d'extraits riches en PACs, en particulier à partir de canneberge, susceptibles d'avoir des effets antibactériens, notamment dans le cadre des infections urinaires. Le procédé décrit et revendiqué implique une étape de lavage à l'eau déminéralisée de la résine utilisée pour enrichir l'extrait en PACs, qui conduit à l'élimination des sucres et des acides phénoliques. De plus, une étape optionnelle visant à éliminer les anthocyanidines est également décrite. Le procédé décrit vise donc là encore avant tout à enrichir au maximum l'extrait en PACs, au détriment des autres composés actifs contenus dans la canneberge.

Ainsi, il a généralement été considéré ces dernières années que l'effet positif de la consommation de canneberge sur la prévention des infections urinaires était lié aux effets anti-adhésion, bactériostatiques et bactéricides des PACs compris dans la canneberge.

Cependant, les PACs sont peu bio-disponibles sous leur forme de PACs natifs, et seulement une faible proportion des PACs semble franchir la barrière intestinale tels quels (Appeldoorn et al., 2009). Les PACs semblent en outre d'autant moins biodisponibles que leur degré de polymérisation est élevé (Ou et al., 2012). Ainsi, la majorité des PACs de canneberge restent dans la lumière intestinale traversant l'intestin grêle pour atteindre le colon. A ce niveau, les PACs faiblement polymérisés sont hydrolysés sous forme d'acides phénoliques et deviennent bio-disponibles sous cette forme.

En conséquence, si les effets anti-adhésion, bactériostatiques et bactéricides des PACs typiques de la canneberge sont avérés in vitro, ils ne peuvent avoir lieu de façon majoritaire dans la vessie ou le tractus urinaire, une très faible proportion de ces composés étant susceptible d'atteindre les voies urinaires. En revanche, ces effets sont possibles dans le colon, mais il n'est pas clair s'ils peuvent expliquer à eux seuls l'effet positif de la consommation de canneberge sur la prévention des infections urinaires.

Il est donc difficile, au vu de l'art antérieur, de déterminer quels composants de la baie de canneberge entière permettent d'éviter ou de diminuer la survenue d'infections urinaires.

Dans le cadre de la présente invention, les inventeurs ont élaboré un nouvel extrait de canneberge qui, au lieu de se focaliser sur un type de composant particulier (PACs, acides phénoliques, acides organiques) susceptible de posséder un effet antibactérien, incorpore dans un unique extrait des fractions enrichies de chacun de ces composants. De plus, l'extrait selon l'invention est un extrait deestérifié, c'est-à-dire qui a été soumis, à une étape ou à une autre, à un traitement enzymatique par des estérases, ce qui a pour effet d'augmenter la concentration en composés d'intérêt et de rendre l'extrait plus fermentescible.

L'extrait selon l'invention possède donc l'avantage d'être fortement concentré en composés d'intérêt par rapport à la baie de canneberge, et de combiner différents mécanismes de prévention ou de diminution de la survenue d'infections urinaires.

### DESCRIPTION DE L'INVENTION

La présente invention concerne donc un extrait de canneberge, comprenant :
- 5 à 20% en poids, avantageusement 10 à 15% en poids, de proanthocyanidols (PACs) par rapport au poids total de l'extrait sec (mesurés par la méthode de BL-DMAC),
- 2 à 12% en poids, avantageusement 3 à 10% en poids, avantageusement strictement plus de 5% et moins de 10% en poids, d'acides organiques par rapport au poids total de l'extrait sec, dont :
   ∘ 1-5% en poids, avantageusement 1-3% en poids d'acide quinique par rapport au poids total de l'extrait sec,
   ∘ 0,5 à 8% en poids, avantageusement 1-4% en poids, d'acides phénoliques par rapport au poids total de l'extrait sec,
- au moins 0,5% en poids, avantageusement au moins 1% en poids, d'anthocyanes et/ou d'anthocyanidols par rapport au poids total de l'extrait sec,
- 1-10%, avantageusement 1-5 % en poids, en poids de sucres par rapport au poids total de l'extrait sec,
- 1-10% en poids, avantageusement 2-5 % en poids, de flavonols, par rapport au poids total de l'extrait sec.

Dans un mode de réalisation préféré de la présente invention, l'extrait de canneberge est en outre désestérifié.

Par « canneberge », on entend la plante *Vaccinium macrocarpon,* appelée également « canneberge d'Amérique » ou « grande airelle rouge d'Amérique », ou encore « atoca » ou « abaca » au Québec, en anglais « cranberry » ou « American cranberry ». Pour simplifier, le terme « canneberge » est généralement utilisé dans la présente description.

Par « désestérifié », on entend que l'extrait a subi, au cours de son procédé de préparation, une étape de traitement enzymatique par au moins une enzyme de type « estérase », capable d'hydrolyser certaines liaisons ester, choisie parmi les tannases (notamment les galloyl-estérases et les ellagi-estérases), les cinnamoylestérases, les B-glucosidases et leurs mélanges. Ce traitement a pour effet d'augmenter la fermentescibilité intestinale de l'extrait selon l'invention, en hydrolysant, au moins partiellement, certains composés complexes présents dans la canneberge. Avantageusement, on peut utiliser un mélange comprenant des activités tannase, cinnamoyl-estérase et B-glucosidase.

Par « tannase », on entend une enzyme capable d'hydrolyser la liaison ester entre le sucre et les acides galliques ou ellagiques des gallotanins et ellagitanins. En particulier, dans la nomenclature EC (EC étant le sigle de « Enzyme Commission numbers », la Commission des enzymes, qui a défini une classification numérique des enzymes, basée sur la réaction chimique qu'elles catalysent) les tannases portent le n°EC 3.1.1.20, et permettent de catalyser les réactions suivantes:
- digallate + H₂O = 2 gallate,
- ellagitannins + H₂O acide hexahydroxydiphenique + acide gallique,
- (+/-)-epicatechine-gallate + H₂O = epicatechine + acide gallique,
- (+/-)-epigallocatechin-3-gallate + H₂O = epigallocatechin + acide gallique,
- (-)-epigallocatechin gallate + H₂O = (-)-epigallocatechin + acide gallique,
- 1,2,3,4,6-pentagalloyl glucose + H₂O = 5 gallate + D-glucose,
- tannic acid + H₂O = 10 gallate + D-glucose,
- propyl gallate + H₂O = gallate + propanol,
- protocatechuic acid ethyl ester + H₂O =protocatechuate + éthanol.

Lorsque des tannases sont utilisées, le traitement a pour effet notamment d'hydrolyser les gallotanins et ellagitanins de la canneberge en sucres et acide gallique ou acide ellagique respectivement, deux acides phénoliques naturellement peu ou pas présents sous forme libre dans la canneberge. De telles enzymes sont disponibles commercialement chez différents fournisseurs tels que :
- AB Enzymes GmbH, Feldbergstrasse 78, 64293 Darmstadt, Germany : (Rohapect 10L; Rohament CL; Rohalase BX/BXL; Endozym B-Split)
- Biocatalysts Ltd, Cefn Coed, Parc Nantgarw, Cardiff, CF15 7QQ, Wales, UK : (Depol 670L, Depol 40, Depol 793L ; Cellulase 13L, Tannase 795P)
- Novozymes A/S, Krogshoejvej 36,2880 Bagsvaerd,Denmark : (Pectinex BE-3L; Pectinex Ultra SP-L)

Par « ß-glucosidases », on entend une enzyme capable d'hydrolyser un glucose non réduit terminal, lié à un aglycone par une liaison B, avec libération de D-glucose. Dans la nomenclature EC, les B-glucosidases portent le n°EC 3.2.1.21, et permettent de catalyser les réactions :
- anthocyanidine-glucoside + H₂O = anthocyanidine + glucose avec la partie anthocyanidine : cyanidine, petunidine, malvidine, peonidine,
   et avec la partie glucoside : glucose, galacatose, arabinose principalement,
- quercetine-3,4'-di-beta-D-glucopyranoside + H₂O quercetine + beta-D-glucose,
- quercetine-4'-beta-glucopyranoside + H₂O quercetine + beta-D-glucose,
- quercetine-7-O-beta-D-glucoside + H₂O quercetine + beta-D-glucose.

De telles enzymes sont disponibles commercialement chez différents fournisseurs, et notamment chez ceux mentionnés ci-dessus concernant les tannases.

Par « cinnamoyl-estérase » on entend les enzymes portant les n° EC 3.1.1.42 et EC 3.1.1.73 dans la nomenclature EC, et capables de catalyser les réactions suivantes :
- EC 3.1.1.42 - chlorogénate hydrolase :
   - acide chlorogénique + H₂O = acide caféique + acide quinique,
   - acide 5-O-caffeoyl quinique + H₂O = acide caféique + acide quinique ;
- EC 3.1.1.73 - féruloyl estérase:
   - acide férulique méthyl ester + H₂O = acide férulique + methanol,
   - 5-O-(trans-féruloyl)-L-arabinofuranoside + H₂O = acide férulique + L-arabinose,
   - acide 5-O-p-coumaroylquinique + H₂O = acide p-coumarique + acide quinique.

De telles enzymes sont disponibles commercialement chez différents fournisseurs, et notamment chez ceux mentionnés ci-dessus concernant les tannases.

Le traitement enzymatique par au moins une estérase permet donc également d'augmenter la concentration de l'extrait notamment en acides organiques et en acides phénoliques libres. Les enzymes décrites plus haut pourront être utilisés tout au long de la chaine de production à des teneurs variables généralement de 0,05% à 0,1% de la matière sèche de canneberge mise en jeu.

Avantageusement, le traitement enzymatique par au moins une enzyme de type « estérase », capable d'hydrolyser certaines liaisons ester, choisie parmi les tannases (notamment les galloyl-estérases et les ellagi-estérases), les cinnamoylestérases, les B-glucosidases et leurs mélanges, a été réalisé simultanément ou à la suite d'un traitement par des enzymes de liquéfaction (pectinase, polygalacturonase, cellulase) visant principalement à :
- distinguer des fractions phénoliques hydrolysables de fractions résistantes à l'hydrolyse,
- rendre ces composés plus apolaires et par conséquent plus facilement séparable.

Certaines préparations comprenant des enzymes de liquéfaction (pectinase, polygalactuonase, cellulase) peuvent comporter minoritairement des activités secondaires du type cellobiase (B-glucosidase), galactosidase, arabinosidases, xylanases, voire des activités secondaires de type tannase, cinnamoyl-estérase, et/ou B-glucosidase, susceptibles de conduire à une désestérification très limitée des polyphénols de canneberge. Cependant, l'étape de liquéfaction enzymatique ne permet pas d'obtenir une désestérification significative de l'extrait, qui ne peut être obtenue que par le biais d'une étape supplémentaire de traitement enzymatique spécifique par au moins une estérase choisie parmi les tannases (notamment les galloyl-estérases et les ellagi-estérases), les cinnamoyl-estérases, les B-glucosidases et leurs mélanges. Par conséquent, un extrait de canneberge ayant subi une simple étape de liquéfaction enzymatique à l'aide d'une composition comprenant très majoritairement des enzymes de liquéfaction de type pectinase, polygalacturonase, et cellulase, sans ajout supplémentaire d'au moins une estérase choisie parmi les tannases (notamment les galloyl-estérases et les ellagi-estérases), les cinnamoylestérases, les B-glucosidases et leurs mélanges, ne sera pas considéré comme un extrait désestérifié au sens de l'invention.

Par « proanthocyanidols », « proanthocyanidines » ou « tannins condensés », abrégés en « PACs », on entend les oligomères ou polymères de flavanols de canneberge. Le terme « oligomère » regroupe les dimères et trimères de flavanols, le terme « polymère » regroupant quant à lui les multimères de flavanols ayant un degré de polymérisation (DP) d'au moins 4. L'extrait désestérifié de canneberge selon l'invention comprend à la fois des oligomères et des polymères de PACs, dans une proportion globale de 5 à 20% en poids, avantageusement 10 à 15% en poids, de PACs par rapport au poids total de l'extrait sec. Au sein des PACs totaux, les polymères de PACs (DP≥4) représentant au moins 30% en poids, par rapport au poids de tous les PACs présents dans l'extrait.

In vivo, les polymères de PACs sont très peu bio-disponibles et restent en majorité dans la lumière intestinale traversant l'intestin grêle pour atteindre le colon, où ils sont susceptibles d'avoir un effet anti-adhésion, bactéricide et bactériostatique sur les bactéries entériques susceptibles de migrer vers l'urètre et de donner lieu à des infections urinaires. La présence de polymères de PACs dans l'extrait selon l'invention contribue donc à son efficacité de prévention ou de diminution des infections urinaires.

Les oligomères de PACs sont également peu bio-disponibles in vivo et parviennent en majorité jusqu'au colon, où ils sont hydrolysés sous forme d'acides phénoliques. Ceux-ci, et notamment les plus apolaires d'entre eux tels que l'acide benzoïque, peuvent alors exercer un autre type d'effet bactérien, par perturbation des possibilités d'échanges métaboliques des transporteurs des membranes bactériennes.

L'extrait selon l'invention comprend également 2-12% en poids, avantageusement 3-10% en poids, avantageusement strictement plus de 5% et moins de 10% en poids, d'acides organiques par rapport au poids total de l'extrait sec. Par « acide organique », on entend tout composé hydrocarboné (saturés ou insaturés) comprenant au moins une fonction acide. Ainsi, les acides organiques de canneberge présents dans l'extrait selon l'invention incluent :
- Des acides phénoliques, dans une quantité de 0,5-8% en poids, avantageusement 1-4% en poids, par rapport au poids total de l'extrait sec.
   L'extrait selon l'invention comprend notamment les principaux acides phénoliques présents naturellement dans la canneberge : l'acide p-coumarique, l'acide sinapique, l'acide caféique et l'acide férulique, ainsi que d'autres acides phénoliques présents en plus faible quantité dans la canneberge : acide ortho-hydroxy-cinnamique, acide para-hydroxyphényl acétique, acide phtalique, et acide ellagique.
   De plus, lorsque l'extrait a subit une étape de traitement par au moins une estérase, les quantités de certains acides phénoliques tels que les acides gallique, ellagique, p-coumarique, férulique et caféique libres sont augmentées par rapport à un extrait n'ayant pas subi ce type de traitement.
   Les acides phénoliques, qui sont capables de passer la barrière intestinale, sont ensuite normalement conjugués avec de la glycine pour former de l'acide hippurique (qui possède un pouvoir bactéricide moindre), avant leur excrétion dans les urines. Cependant, du fait des quantités assez importantes d'acides phénoliques présentes dans l'extrait selon l'invention (et en particulier dans un extrait désestérifié ayant subi un traitement enzymatique par des estérases), il n'est pas impossible que les capacités de conjugaison avec la glycine par le foie soient saturées et qu'une partie des acides phénoliques, non-conjugués à de la glycine, puissent atteindre l'urine et y exercer leurs effets bactéricides de manière préventive ou curative. La présence dans l'extrait de différents précurseurs d'acides phénoliques (notamment l'acide quinique, qui peut être transformé en acide benzoïque par aromatisation réductrice ; les oligomères de PACs, qui sont hydrolysés sous forme d'acides phénoliques dans le colon ; et les anthocyanes ou anthocyanidols hydrolysés sous forme d'acides benzoïques dans le colon) contribue également à la présence dans l'organisme d'une concentration élevée et soutenue en acides phénoliques, et donc à une saturation des capacités de conjugaison avec la glycine par le foie.
- D'autres acides organiques naturellement présents dans la canneberge, en particulier l'acide quinique, l'acide citrique, et l'acide malique.
   L'extrait selon l'invention comprend notamment 1-5% en poids, avantageusement 1-3% en poids d'acide quinique par rapport au poids total de l'extrait sec.
   L'acide quinique est réduit en acide benzoïque (un acide phénolique) au niveau du colon par le matériel enzymatique de la flore intestinale. L'acide benzoïque ainsi obtenu au niveau du colon n'est pas métabolisé et peut donc exercer ses effets bactéricides sur les bactéries présentes dans le colon et susceptibles de migrer dans l'urètre et de causer une infection urinaire.
   Par conséquent, la présence dans l'extrait selon l'invention (et en particulier dans un extrait désestérifié ayant subi un traitement enzymatique par des estérases) d'une quantité non négligeable d'acide quinique contribue par un troisième mécanisme au pouvoir de prévention ou de diminution des infections urinaires de l'extrait selon l'invention.

L'extrait selon l'invention comprend également au moins 0,5 % en poids, avantageusement au moins 1 % en poids, d'anthocyanes et/ou d'anthocyanidols par rapport au poids total de l'extrait sec. Par « anthocyanidol » ou « anthocyanidine », on entend une sous-classe des flavonoïdes, dont la structure de base est formée de deux noyaux aromatiques A et B joint par 3 carbones formant avec l'oxygène le cycle C. Les six anthocyanidols les plus courants sont: la cyanidine, la delphinidine, la pélargonidine, la péonidine, la pétunidine et la malvidine, construits sur le même squelette flavylium responsable de la couleur du composé, comme indiqué dans le **Tableau 1** ci-dessous :

**Tableau 1. Structure des six anthocyanidols les plus courants.**

| **Nom** | **Structure** | **R**^{**3**'} | **R**^{**4**'} | **R**^{**5**'} | **R³** | **R⁵** | **R⁶** | **R⁷** |
|---|---|---|---|---|---|---|---|---|
| Cyanidine | | -OH | -OH | -H | -OH | -OH | -H | -OH |
| Delphinidine | 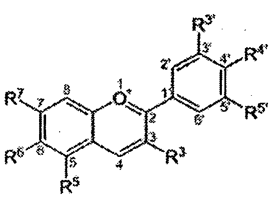 | -OH | -OH | -OH | -OH | -OH | -H | -OH |
| Pélargonidine | | -H | -OH | -H | -OH | -OH | -H | -OH |
| Malvidine | | -OCH₃ | -OH | -OCH₃ | -OH | -OH | -H | -OH |
| Péonidine | | -OCH₃ | -OH | -H | -OH | -OH | -H | -OH |
| Pétunidine | | -OCH₃ | -OH | -OH | -OH | -OH | -H | -OH |

Par « anthocyanes » ou « anthocyanosides » (ou encore « anthocyanines » sur le modèle anglais), on entend des hétérosides d'anthocyanidols, c'est-à-dire des anthocyanidols portant des sucres. La partie osidique des anthocyanosides peut être un monosaccharide (glucose, galactose, rhamnose), un diholoside (rutinose constitué d'un glucose lié à un rhamnose, xyloglucose) ou parfois un triholoside. La plupart des anthocyanosides sont des 3-monosides et des 3, 5-diosides d'anthocyanidols. Il existe aussi des diosides liés en 3, 7 et des triosides liés en 3, 5, 3'. De nombreux anthocyanosides sont en outre acylés par :
- des acides hydroxycinnamiques : acides 4-coumarique, caféique, férulique, sinapique,
- des acides benzoïques : acide gallique,
- des acides aliphatiques carboxyliques : acide acétique, ou des acides dicarboxyliques comme les acides malonique, malique, oxalique, succinique.

Ces acides estérifient un hydroxyle de sucre, généralement sur leur C-6.

Lorsque l'extrait n'a pas été désestérifié, il comprend principalement des anthocyanes. En revanche, lorsque l'extrait a été désestérifié, il comprend principalement des anthocyanidols.

Les anthocyanes, et plus particulièrement les anthocyanidols (présents majoritairement lorsque l'extrait est désestérifié), ont été montrés avoir également des activités bactéricides (Pratt et al., 1960). De plus, ils sont également fermentés par la flore intestinale, conduisant au niveau du colon à leur transformation en acides phénoliques, et notamment en acide benzoïque (Keppler et al., 2005). Ces composés sont donc également des précurseurs d'acides phénoliques in vivo.

L'extrait selon l'invention comprend en outre 1-10%, avantageusement 1-5% en poids de sucres résiduels par rapport au poids total de l'extrait sec. Cela représente une forte diminution de la teneur en sucres par rapport à la baie de canneberge, dans laquelle les sucres représentent environ 45 à 67 % (Blumenthal et al, 2003) en poids de sucres par rapport au poids total de l'extrait sec. Les sucres présents incluent notamment du glucose, fructose, saccharose, sorbitol.

L'extrait selon l'invention comprend également 1-10% en poids, avantageusement 2-5% en poids, de flavonols, tels que la quercétine, sous forme glycosylée et/ou aglycone, par rapport au poids total de l'extrait sec.

Ainsi, l'extrait selon l'invention possède une composition complexe, qui permet de cumuler plusieurs effets antibactériens au niveau du colon (effets anti-adhésion, bactéricide et bactériostatique via les polymères de PACs présents dans le colon, effets bactéricides des acides phénoliques générés au niveau du colon à partir des oligomères de PACs et de l'acide quinique), ces effets synergisants pour conférer à l'extrait selon l'invention un fort pouvoir de prévention ou de diminution de l'occurrence des infections urinaires.

De plus, la présence d'acides phénoliques en quantités importantes permet potentiellement, par le biais d'une saturation des capacités du foie à conjuguer les acides phénoliques avec la glycine, un effet bactéricide direct au niveau des voies urinaires, de façon préventive ou curative.

L'extrait selon l'invention peut se présenter sous différentes formes, en particulier sous forme de solution, ou bien sous forme sèche. Avantageusement, l'extrait selon l'invention est un extrait sec. Sous forme de poudre, celui-ci peut être utilisés sous différentes formes, permettant par exemple de préparer, des comprimés, des gélules, de granules et des solutions orodispersibles. Sous forme solide ou liquide il peut aussi permettre de formuler des gels, crèmes, savons pour des applications topiques, et de formuler des boissons diluées à reconstituer ou prêtes à l'emploi.

L'extrait selon l'invention peut être préparé par un procédé selon l'invention comprenant les étapes suivantes :
a) Enrichissement d'une fraction hydrosoluble de canneberge en composés polyphénoliques apolaires (anthocyanes, anthocyanidols, et oligomères de PACs), d'une part, et en acides organiques, d'autre part, pour obtenir une fraction I,
b) Enrichissement d'une fraction non-hydrosoluble de canneberge en polymères de PACs particulièrement tannants avec des degrés de polymérisation moyen supérieur à 6-7 de type B et comportant toujours au moins une liaison de type A, pour obtenir une fraction II,
c) Assemblage des fractions I (riche en composés polyphénotiques apolaires et en acides organiques) et **II** (riche en polymères de PACs) obtenues respectivement aux étapes a) et b), dans des proportions correspondant à un rapport en poids (fraction **I**/fraction **II**) compris entre 20/80 et 80/20, et
d) Digestion enzymatique par au moins une estérase choisie parmi les tannases (notamment les galloyl-estérases et les ellagi-estérases), les cinnamoyl-estérases, les B-glucosidases et leurs mélanges.

Dans le procédé selon l'invention, l'extrait est préparé à partir de la combinaison de deux fractions **I** et **II**, obtenues respectivement à partir de fractions hydrosoluble (pour la fraction **I**) et non-hydrosoluble (pour la fraction **II**) de canneberge.

Ces fractions hydrosoluble et non-hydrosoluble de canneberge peuvent être obtenues à partir de baies de canneberge entières, par :
i) Broyage de baies de canneberge entières,
ii) Décantation et séparation des fractions hydrosoluble et non-hydrosoluble.

Les baies de canneberge entières peuvent être fraîches, sèches ou surgelées (de préférence par un procédé de congélation individuelle rapide dit « IQF » (pour « Individually Quick Frozen »)). Elles sont ensuite broyées par des techniques classiques (étape i)). A ce stade, le broyat est de préférence soumis à une étape de liquéfaction enzymatique (étape i1)). Cette étape est une étape classique bien connue de l'homme du métier, pendant laquelle des pectinases, polygalacturonases et/ou cellulases sont ajoutées au broyat de façon à le liquéfier. Le broyat, de préférence liquéfié, est ensuite soumis à une étape de décantation et de séparation des fractions hydrosoluble et non-hydrosoluble (étape ii)). Cette étape est avantageusement réalisée à un pH compris entre 2 et 7, à une température comprise entre 15 et 70°C, et à une pression comprise entre 1 et 15 atmosphères.

De manière alternative, on peut directement utiliser comme fraction hydrosoluble un jus de canneberge, c'est-à-dire une fraction liquide de canneberge obtenue par pressage de baies de canneberge. Le jus peut en outre être un jus concentré. Celui-ci a de préférence été soumis à une étape de liquéfaction enzymatique par au moins une pectinase, polygalacturonase, cellulase et leurs mélanges.

De même, on peut directement utiliser comme fraction non-hydrosoluble un marc de canneberge. Par « marc de canneberge » ou « drèches de canneberge », on entend le résidu humide ou séché de baies de canneberge pressées. Ceux-ci ont de préférence été soumis à une étape de liquéfaction enzymatique par au moins une pectinase, polygalacturonase, cellulase et leurs mélanges.

Dans le procédé selon l'invention, les fractions hydrosoluble et non-hydrosoluble sont traitées séparément (étape a) pour la fraction hydrosoluble, étape b) pour la fraction non-hydrosoluble). Ces traitements séparés peuvent être réalisés dans un ordre quelconque (étape a) puis étape b), étape b) puis étape a), ou traitement simultané en parallèle des deux fractions).

A l'étape a), la fraction hydrosoluble est enrichie en composés polyphénoliques apolaires, d'une part, et en acides organiques, d'autre part, pour obtenir une fraction appelée « fraction **I** ». Cet enrichissement est réalisé par deux étapes successives de purification, suivies d'une étape d'assemblage conduisant à la fraction **I**:
- Dans une 1^{ère} étape a1), les composés apolaires (et notamment les composés phénoliques apolaires, comme les flavanols, flavonols et les anthocyanes, éventuellement désestérifiés) sont séparés des composés polaires (tels que les sucres et les acides) par adsorption de la fraction hydrosoluble sur un support apolaire. Un lavage par un solvant polaire permet de collecter les composés polaires non adsorbés sur le support (fraction appelée « fraction polaire la »). Une étape d'élution par un solvant apolaire permet ensuite de collecter les composés apolaires (fraction appelée « fraction apolaire Ib »).
   Les supports apolaires adaptés à cette séparation incluent par exemple :
   ∘ les supports d'adsorption de type copolymère styrène / divinyl benzène, sulfoné ou non :
      ▪ FPX68 ; FPX66 (Dow Chemicals)
      ▪ Sepabeads SP411, SP70, SP700, SP852L, SP850 (Mitsubishi Chemicals) ;
   ∘ les supports microporeux supportant des groupes ionisés de type sulfone ou amine tertiaire.
      ▪ FPA51, FPA54, FPC23H, XAD761 (Dow Chemicals),
      ▪ Diaion UBK530, UBK550, WA20, WA30.

   Les solvants polaires de lavage permettant de récupérer la fraction polaire la peuvent notamment être choisis parmi les alcools (en particulier l'éthanol, le méthanol, le 2-propanol).
   Les solvants apolaires d'élution permettant de récupérer la fraction apolaire Ib selon la résine échangeuse d'ions choisie peuvent notamment être choisis parmi des solutions diluées de soude, de potasse, ou des solutions diluées d'acide fort acide chlorhydrique, sulfurique ou faible d'acide citrique.
   A l'issue de cette étape a1), la fraction apolaire Ib comprenant notamment les composés phénoliques apolaires tels que les anthocyanes ou anthocyanidols et les oligomères de PACs est mise de côté, tandis que la fraction polaire la comprenant notamment les sucres et les acides organiques est soumise à une deuxième étape a2) de purification.
- Dans une 2^{ème} étape a2), les acides organiques présents dans la fraction polaire la sont purifiés (et notamment séparés des sucres également présents dans la fraction polaire la) par adsorption sur une résine échangeuse d'ions (qui retient les acides organiques, mais pas les sucres). Après lavage avec de l'eau (ce qui conduit notamment à une élimination de la plupart des sucres), une fraction appelée « fraction **I**c » enrichie en acides organiques de canneberge (acide quinique, acide citrique, acide malique et acides phénoliques notamment) est éluée à l'aide d'un solvant aqueux acide.
- Dans une 3^{ème} étape a3), la fraction **I**, enrichie en composés polyphénoliques apolaires (anthocyanes, anthocyanidols, et oligomères de PACs), d'une part, et en acides organiques, d'autre part, est obtenue par assemblage des fractions Ib et Ic.

A l'étape b), la fraction non-hydrosoluble (marc) est enrichie en polymères de PACs, pour obtenir une fraction appelée « fraction II », par un traitement comprenant une étape d'extraction des polymères de PACs par un solvant organique, de préférence suivie d'étapes de clarification, distillation et concentration de la solution enrichie ainsi obtenue. Différents procédés de ce type sont décrits dans la demande EP2108268A1.

Ainsi, le solvant organique utilisé pour l'extraction peut en particulier être choisi parmi les alcools (notamment l'éthanol), les aldéhydes (notamment l'acétaldéhyde), et les esters (notamment acétate d'éthyle). Avantageusement, le solvant organique utilisé est un alcool, en particulier l'éthanol.

La quantité de solvant organique ajoutée à la fraction hydrosoluble de canneberge peut notamment être comprise entre 10 et 15% en volume, par rapport au volume de la fraction hydrosoluble de canneberge sur laquelle l'extraction est réalisée.

En particulier, l'étape d'extraction peut être réalisée par extraction hydroalcoolique à contre-courant, les mélanges hydro-alcooliques comportant de 35 à 80% d'éthanol sont déversés successivement sur des parts de marcs de canneberge frais de moins en moins épuisées ; les fractions éthanoliques comportant quant à elles de plus en plus de matière sèche extractible solution.

Des étapes finales de clarification, distillation et concentration sont avantageuses.

Les fractions I (Ib et **I**c enrichies en composés phénoliques apolaires tels que anthocyanes, anthocyanidols, et oligomères de PACs, et en acides organiques) et **II** (enrichie en polymères de PACs) sont ensuite assemblées pour former un extrait enrichi en ces différents composés d'intérêt. L'assemblage peut être réalisé dans des proportions variables correspondant à un rapport en poids (fraction **I**/fraction **II**) compris entre 20/80 et 80/20, avantageusement entre 30/70 et 70/30, entre 35/65 et 65/35, entre 40/60 et 60/40, voire entre 45/55 et 55/45. Avantageusement, le rapport en poids (fraction **I**/fraction **II**) peut être proche de 50/50. En effet, cela correspond à un assemblage dans lequel les fractions **I** et **II** obtenues respectivement à l'issue des étapes c) et d), sont simplement mélangées, sans élimination d'une partie de l'une des fractions. Ainsi, l'ensemble des composés d'intérêt est valorisé dans l'extrait, sans perte liée à l'exclusion d'une partie des composés d'intérêt. Cependant, d'autres rapports en poids (fraction **I**/fraction **II**) peuvent être utilisés si l'on souhaite augmenter ou diminuer la proportion dans l'extrait de certains composés d'intérêt.

Le procédé selon l'invention de préparation de l'extrait selon l'invention comprend en outre une étape d) de digestion enzymatique par au moins une estérase. Cette étape d) vise à augmenter la fermentescibilité intestinale de l'extrait selon l'invention, en hydrolysant, au moins partiellement, certains composés complexes présents dans la canneberge, et n'interfère pas de façon significative avec les autres étapes du procédé de préparation. Elle peut par conséquent être réalisée à différents stades du procédé de préparation. En particulier, elle peut être réalisée :
- avant l'étape a), au cours de la préparation des fractions hydrosoluble et non-hydrosoluble (par exemple entre les étapes i) et ii), sur le broyat, de préférence liquéfié), ou bien sur les fractions hydrosoluble et non-hydrosoluble, de préférence liquéfiées,
- entre les étapes a) et b) et l'étape c) d'assemblage final, sur les fractions **I** et **II**, ou
- à la fin du procédé, sur l'extrait assemblé obtenu à l'issue de l'étape c).

Le procédé selon l'invention peut en outre comprendre une étape additionnelle de séchage de l'extrait, de façon à obtenir un extrait sec. Un tel séchage peut être réalisé par toute technique classique adaptée, et notamment par atomisation, à l'aide d'une tour de séchage simple ou multi-effets alimenté par des concentrés aqueux présentant une matière sèche supérieure à 20% et préalablement mis à température (50°-80°C). Ces concentrés sont pulvérisés par des systèmes de buse et/ou de turbines et séchés sous forme de poudre à partir de gouttelettes nébulisées dans un flux d'air sec à des températures inférieures à 180°C. Selon les cas, les fractions polyphénoliques des extraits de canneberge peuvent être séchées sans support dans le cas des extraits apolaires développés, ou avantageusement sur des supports tels que des carbohydrates, (maltodextrine, amidons résistants, inuline). Dans le cas des fractions particulièrement acides, les fractions peuvent être neutralisées par ajout d'hydroxyde de magnésium avant séchage.

L'invention concerne également un extrait susceptible d'être obtenu par le procédé selon l'invention. Un tel extrait comprend de préférence :
- 5 à 20% en poids, avantageusement 10 à 15% en poids, de proanthocyanidols (PACs) par rapport au poids total de l'extrait sec,
- 2-12% en poids, avantageusement 3-10% en poids, avantageusement strictement plus de 5% et moins de 10% en poids, d'acides organiques par rapport au poids total de l'extrait sec, dont :
   ∘ 1-10% en poids, avantageusement 1-5% en poids, avantageusement 1-3% en poids d'acide quinique par rapport au poids total de l'extrait sec,
   ∘ 0,5-8% en poids, avantageusement 1-4 % en poids, d'acides phénoliques par rapport au poids total de l'extrait sec,
- au moins 0,5% en poids, avantageusement au moins 1 % en poids, d'anthocyanes par rapport au poids total de l'extrait sec,
- 1-10%, avantageusement 1-5% en poids, en poids de sucres par rapport au poids total de l'extrait sec,
- 1-10% en poids, avantageusement 2-5% en poids, de flavonols, par rapport au poids total de l'extrait sec.

L'extrait susceptible d'être obtenu par le procédé selon l'invention peut également se présenter sous différentes formes, en particulier sous forme de solution (notamment sous forme de concentré liquide), ou bien sous forme sèche. Avantageusement, l'extrait susceptible d'être obtenu par le procédé de l'invention est un extrait sec. Celui-ci peut se présenter sous différentes formes, par exemple sous forme de poudre, de comprimé, de gélule, de granules.

L'invention concerne également une composition alimentaire ou nutraceutique comprenant, entre autres, un extrait selon l'invention ou un extrait susceptible d'être obtenu par le procédé selon l'invention. Cette composition est destinée à être ingérée, et ne comprend donc que des composants acceptables pour cette application. Elle peut se présenter sous toute forme habituellement utilisée en alimentaire ou nutraceutique, et notamment sous forme de poudre, de gélule, de comprimé, de boisson, ou de formules topiques de type gels, crèmes ou savons.

Les extraits selon l'invention comprennent différents composés de canneberge ayant in vivo des effets antibactériens au niveau du colon :
Ils comprennent également des acides phénoliques en quantité importante, susceptible de saturer les capacités du foie à conjuguer les acides phénoliques avec la glycine, cela permettant potentiellement à une partie de ces acides phénoliques d'atteindre les voies urinaires et d'exercer leurs effets bactéricides de façon préventive ou curative.

Les extraits selon l'invention sont donc particulièrement utiles pour la prévention et/ou le traitement des infections urinaires.

L'invention concerne donc également une composition pharmaceutique, comprenant un extrait selon l'invention ou un extrait susceptible d'être obtenu par le procédé selon l'invention et un excipient pharmaceutiquement acceptable.

L'invention concerne en outre un extrait selon l'invention ou un extrait susceptible d'être obtenu par le procédé selon l'invention, pour son utilisation en tant que médicament.

L'invention concerne également un extrait selon l'invention ou un extrait susceptible d'être obtenu par le procédé selon l'invention, pour son utilisation dans la prévention ou le traitement des infections urinaire.

L'invention concerne aussi l'utilisation d'un extrait selon l'invention ou d'un extrait susceptible d'être obtenu par le procédé selon l'invention, pour la préparation d'un médicament, en particulier d'un médicament destiné au traitement ou à la prévention des infections urinaires.

L'invention concerne en outre une méthode de traitement ou de prévention des infections urinaires chez un sujet (de préférence humain, éventuellement animal), comprenant l'administration (de préférence par voie orale) d'une quantité efficace d'un extrait selon l'invention ou d'un extrait susceptible d'être obtenu par le procédé selon l'invention audit sujet.

Par « traitement », on entend le fait de diminuer l'infection bactérienne au niveau des voies urinaires. Une telle diminution peut être mise en évidence par le biais d'analyse d'urine montrant une diminution du nombre de bactéries présentes ou par une diminution des symptômes de l'infection urinaire (envie fréquent d'uriner, brûlures associées au fait d'uriner...). Avantageusement, le traitement permet d'éliminer complètement l'infection, mais le terme « traitement » recouvre toute diminution significative de l'infection. Dans le cadre du traitement des infections urinaires, l'extrait selon l'invention ou l'extrait susceptible d'être obtenu par le procédé selon l'invention peut être associé à un autre traitement usuel des infections urinaires, notamment par différents antibiotiques bien connus de l'homme du métier.

Par « prévention », on entend le fait de diminuer la probabilité de survenue d'une infection urinaire. Avantageusement, l'extrait selon l'invention ou l'extrait susceptible d'être obtenu par le procédé selon l'invention permet d'éviter toute infection urinaire pendant la durée de prise de l'extrait (probabilité de survenue d'infection urinaire valant zéro). Cependant, le terme « prévention » recouvre également la possibilité de diminuer significativement la fréquence de survenue d'infections urinaires dans une population de patients ingérant une quantité efficace d'extrait pendant la durée de prise de l'extrait, par rapport à une population de patients similaires ne prenant pas de l'extrait (auquel cas la probabilité d'infection urinaire pendant la prise de l'extrait est simplement diminuée significativement). Pour une telle comparaison, les populations comparées doivent être similaires, notamment concernant la proportion de sujets ayant usuellement une fréquence élevée de survenue d'infections urinaires.

L'extrait selon l'invention ou l'extrait susceptible d'être obtenu par le procédé selon l'invention sont particulièrement utiles pour la prévention ou le traitement des infections urinaires par les bactéries uropathogéniques : entérobactéries, coliformes, *Escherichia coli* (plus particulièrement E. coli dite « mannose résistante » présentant des adhésines fimbriae de type **I**), mais aussi plus généralement les bactéries gram positives (et notamment *Pseudomonas aeruginosa*), et d'autres bactéries de type *Proteus mirabilis,* les espèces du genre *Staphylococcus* (notamment *Staphylococcus aureus*), les espèces du genre *Saprophyticus,* et les espèces du genre *Klebsiella*. Dans un mode de réalisation, l'extrait selon l'invention ou l'extrait susceptible d'être obtenu par le procédé selon l'invention peut être utilisé dans pour la prévention ou le traitement des infections urinaires par *Escherichia coli.* Dans un autre mode de réalisation, l'extrait selon l'invention ou l'extrait susceptible d'être obtenu par le procédé selon l'invention peut être utilisé dans pour la prévention ou le traitement des infections urinaires par *Pseudomonas aeruginosa*.

Dans encore un autre mode de réalisation, l'extrait selon l'invention ou l'extrait susceptible d'être obtenu par le procédé selon l'invention peut être utilisé dans pour la prévention ou le traitement des infections urinaires par des champignons, et notamment par les espèces du genre *Candida,* particulièrement *Candida albicans*.

L'extrait selon l'invention ou l'extrait susceptible d'être obtenu par le procédé selon l'invention sont particulièrement utiles dans la prévention des récidives d'infections urinaires, chez des patients souffrant d'infections urinaires récurrentes plus ou moins fréquentes.

L'extrait selon l'invention ou l'extrait susceptible d'être obtenu par le procédé selon l'invention peut en particulier être administré à des patients en cours de guérison d'une infection urinaire, afin de diminuer la probabilité de récidive. L'extrait est alors administré dans des quantités efficaces pendant quelques jours (par exemples 3, 4, 5, 6, ou 7 jours) à plusieurs semaines (par exemple 2, 3, 4, 5 ou 6 semaines).

Par « quantité efficace », on entend une quantité permettant d'obtenir un effet traitant ou préventif (tel que défini ci-dessus) chez le sujet d'intérêt. Une quantité efficace d'extrait sec selon l'invention peut notamment être comprise entre 100 et 1000 mg/kg/jour, avantageusement entre 150 et 450 mg/kg/jour.

### DESCRIPTION DES FIGURES

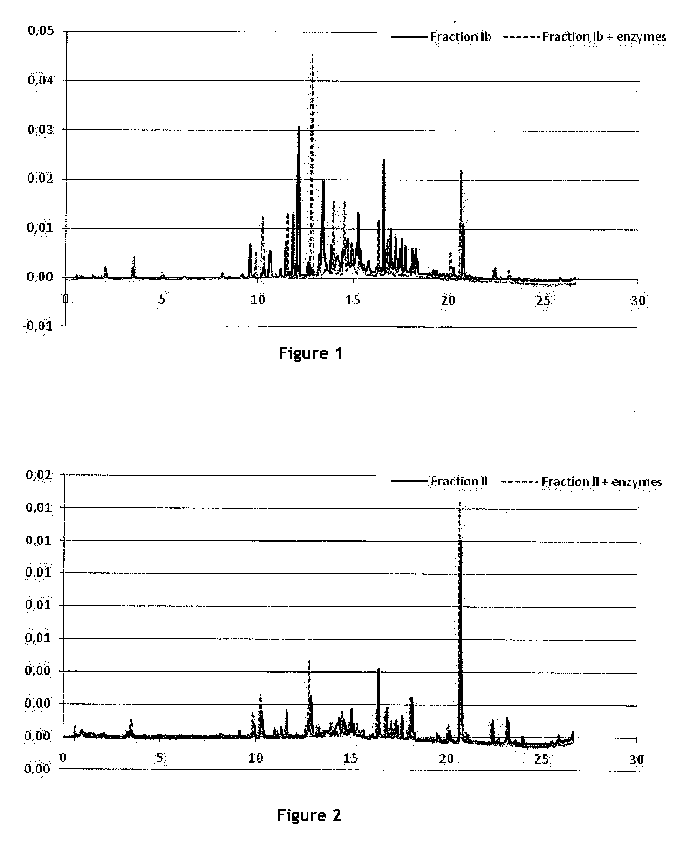

**Figure 1** **:** Profils chromatographiques en UPLC en phase inverse à 280 nm des fractions **I**b obtenues avec ou sans enzymage préalable (Pectinex Ultra SP-L; Cellualse 13L ; Tannase 795P ; Endozym B-split) à 0,05% de la matière sèche mise en jeu dans le broyat de canneberge.
**Figure 2** **:** Profils chromatographiques en UPLC en phase inverse à 280 nm des fractions **II** obtenues avec ou sans enzymage préalable (Pectinex Ultra SP-L; Cellulase 13L ; Tannase 795P ; B-split) à 0,05% de la matière sèche mise en jeu dans le broyat de canneberge.

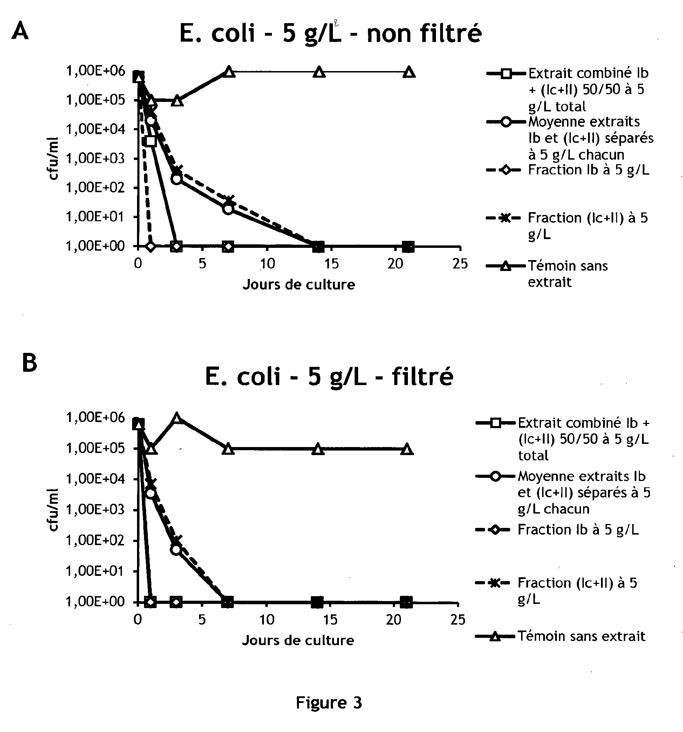

**Figure 3****.** Effets bactéricides de différents extraits sur *Escherichia coli* (ATCC 8739) : croissance des bactéries *Escherichia coli* (ATCC 8739) dans un milieu de culture en absence d'extrait (Témoin sans extrait), en présence de la fraction **I**b à 5 g/L, de la fraction (**I**c+**II**) à 5 g/L, ou du mélange de la fraction **I**b et de la fraction (**I**c+**II**) à 5g/L total (soit 2,5 g/L de fraction **I**b et 2,5 g/L de fraction (**I**c+**II**), Extrait combiné), pour un comptage sans filtration (A) ou après filtration des agrégats (B). Les résultats correspondant à la moyenne des résultats obtenus avec chacune des fractions **I**b, d'une part, et (**I**c+**II**) d'autre part, sont également indiqués.

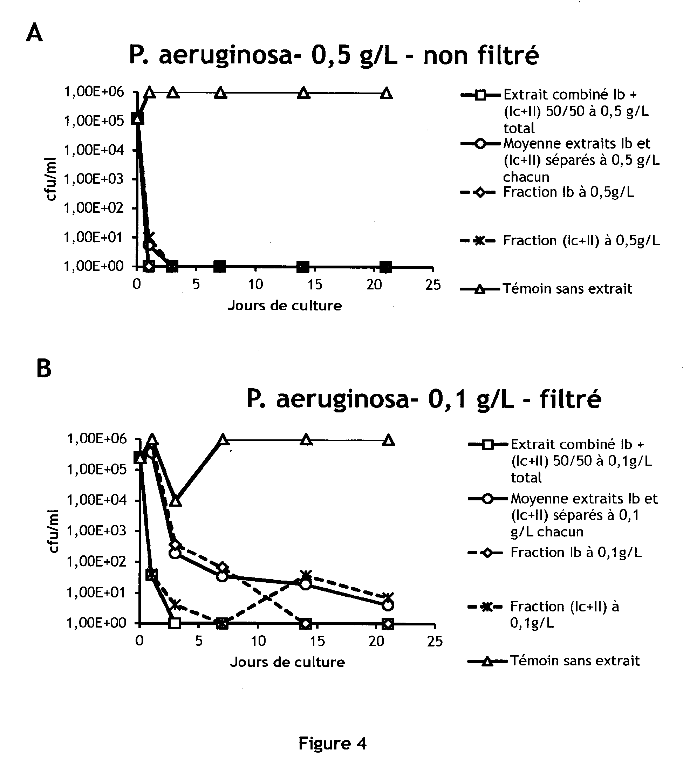

**Figure 4****.** Effets bactéricides de différents extraits sur *Pseudomonas aeruginosa* (ATCC 9027) : (A) Croissance des bactéries *Pseudomonas aeruginosa* (ATCC 9027) dans un milieu de culture en absence d'extrait (Témoin sans extrait), en présence de la fraction Ib à 0,5 g/L, de la fraction (**I**c+**II**) à 0,5 g/L, ou du mélange de la fraction Ib et de la fraction (**I**c+**II**) à 0,5 g/L total (soit 0,25 g/L de fraction Ib et 0,25 g/L de fraction (Ic+II), Extrait combiné), pour un comptage sans filtration. Les résultats correspondant à la moyenne des résultats obtenus avec chacune des fractions **I**b, d'une part, et (**I**c+**II**) d'autre part, sont également indiqués. (B) Croissance des bactéries *Pseudomonas aeruginosa* (ATCC 9027) dans un milieu de culture en absence d'extrait (Témoin sans extrait), en présence de la fraction Ib à 0,1 g/L, de la fraction (**I**c+**II**) à 0,1 g/L, ou du mélange de la fraction Ib et de la fraction (**I**c+**II**) à 0,1 g/L total (soit 0,05 g/L de fraction Ib et 0,05 g/L de fraction (Ic+II), Extrait combiné), pour un comptage après filtration des agrégats. Les résultats correspondant à la moyenne des résultats obtenus avec chacune des fractions **I**b, d'une part, et (**I**c+**II**) d'autre part, sont également indiqués.

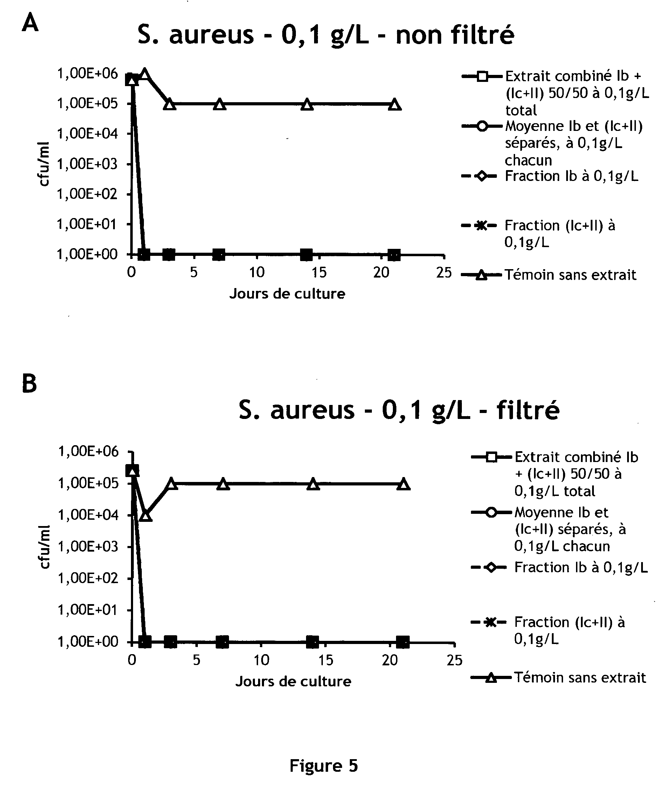

**Figure 5****.** Effets bactéricides de différents extraits sur *Staphylococcus aureus* (ATCC 6538) : croissance des bactéries *Staphylococcus aureus* (ATCC 6538) dans un milieu de culture en absence d'extrait (Témoin sans extrait), en présence de la fraction Ib à 0,1 g/L, de la fraction (**I**c+**II**) à 0,1 g/L, ou du mélange de la fraction **I**b et de la fraction (**I**c+**II**) à 0,1 g/L total (soit 0,05 g/L de fraction **I**b et 0,05 g/L de fraction (**I**c+**II**), Extrait combiné), pour un comptage sans filtration (A) ou après filtration des agrégats (B). Les résultats correspondant à la moyenne des résultats obtenus avec chacune des fractions **I**b, d'une part, et (**I**c+**II**) d'autre part, sont également indiqués.

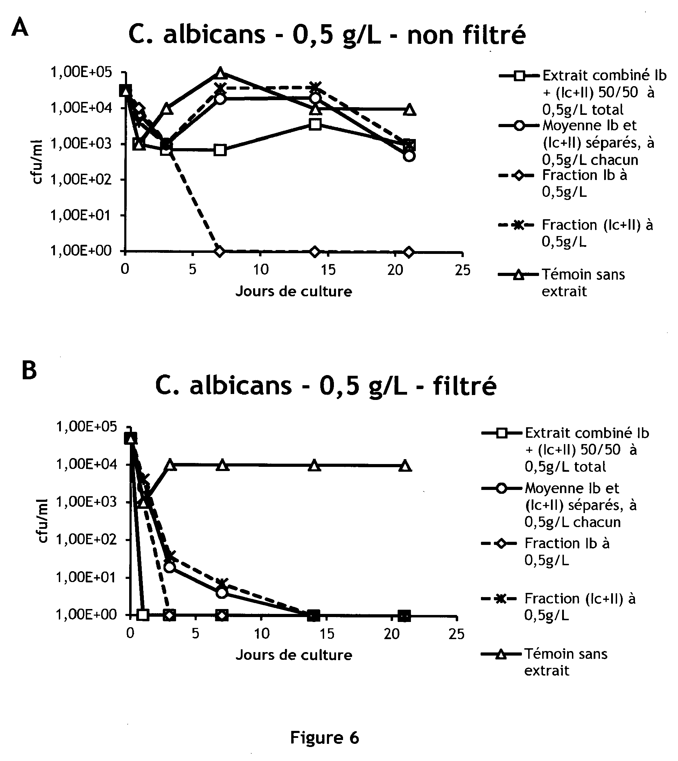

**Figure 6****.** Effets fongicides de différents extraits sur *Candida albicans* (ATCC 10231) : croissance des champignons *Candida albicans* (ATCC 10231)dans un milieu de culture en absence d'extrait (Témoin sans extrait), en présence de la fraction Ib à 0,5 g/L, de la fraction (**I**c+**II**) à 0,5 g/L, ou du mélange de la fraction Ib et de la fraction (**I**c+**II**) à 0,5 g/L total (soit 0,25 g/L de fraction Ib et 0,25 g/L de fraction (Ic+II), Extrait combiné), pour un comptage sans filtration (A) ou après filtration des agrégats (B). Les résultats correspondant à la moyenne des résultats obtenus avec chacune des fractions **I**b, d'une part, et (**I**c+**II**) d'autre part, sont également indiqués.

### EXEMPLES

### Exemple 1. Préparation d'un extrait désestérifié de canneberge selon l'invention,

5 kg de canneberge IQF ont été broyés à l'aide d'un broyeur à marteau et les 400g de matière sèche de broyat ainsi obtenu ont été dilués par 2. La masse de broyat a été divisée par deux et soit traitée directement soit soumise à un traitement enzymatique dans l'eau né d'un mélange d'enzymes comprenant :
∘ 0,05 g de Pectinex Ultra-SP L,
∘ 0,05 g de tannase 795P,
∘ 0,05 g de cellulase 13L, et
∘ 0,05 g de Endozym B-Split.

Le mélange a été porté à 40°C pendant 4h, sous agitation.

Les broyats ont ensuite été pressés et les 2 jus obtenus ont été clarifiées par centrifugation (3000 rpm, 10min) et les surnageants stockés au froid. Les culots de centrifugation ont été ajoutés aux deux marcs de canneberge obtenus parallèlement.

Les marcs ont ensuite été extraits avec un mélange hydroalcoolique à 75% d'éthanol v/v pour un ratio de 3 pour 1, la solution hydro-éthanolique ainsi obtenues a été filtrée sur un filtre PTFE de 0,2 µm de porosité, puis concentrés sous-vide à température modérée (50°C) afin d'obtenir une suspension concentrée qui a été séchée par lyophilisation.

Les fractions de jus obtenues ont été passées sur une colonne d'adsorption remplie avec 200 ml de résine Sepabeads SP411 (BV), à la vitesse de 600 mL/h ; le reste de jus est ensuite lavé avec 800 mL d'eau et la colonne est enfin élué avec 400 ml d'éthanol. L'éluat obtenu est finalement concentré par évaporation sous-vide (50°C) et repris dans l'eau et lyophilisé pour former les fractions la ayant subi une hydrolyse enzymatique ou non.

Les extraits aqueux partiellement décolorés (Fraction Ib) ont ensuite été passés sur une résine échange d'anions remplie de 200 mL de résine FPA51 préalablement mise sous sa forme chlorure avec 400 mL d'acide chlorhydrique 0,1% et rincée à l'eau. Les fractions Ib ont été passées sur la résine à 600 mL/h et lavées avec 400 mL d'eau. Les acides organiques retenus ont été élués avec 400mL l'acide chlorhydrique à 0,05%, puis concentrés sous vide formant la fraction Ic.

### Exemple 2. Caractérisation de l'extrait selon l'invention

L'extrait produit à l'Exemple 1 a été caractérisé.

Les profils chromatographiques en UPLC en phase inverse à 280 nm des fractions Ib et II obtenues avec ou sans enzymage préalable (Pectinex Ultra SP-L; Cellualse 13L ; Tannase 795P ; B-split) à 0,05% de la matière sèche mise en jeu dans le broyat de canneberge sont présentés respectivement sur les **Figures 1** et **2****.**

Ces figures montrent clairement l'effet de la dé-estérification (traitement par au moins une estérase) sur la modification de composition de l'extrait.

Les analyses des différentes fractions d'intérêt obtenues sont décrites dans le **Tableau 2** suivant. Les fractions phénoliques Ib et II peuvent être avantageusement complétées par les acides organiques de l'éluat Ic.

**Tableau 2. Caractérisation des fractions desestérifiées obtenues avec utilisations d'estérases lors de l'extraction**

| en g/100g | | Fraction Ib | Fraction Ic | Fraction II |
|---|---|---|---|---|
| Acides organiques | | | | |
| Ac malique | | 0,11% | 11,88% | 1,93% |
| Ac quinique | | 0,25% | 33,26% | 3,17% |
| Ac citrique | | 0,18% | 14,21% | 3,12% |
| Acides phénoliques | | | | |
| ac gallique | | 0,07% | 0,59% | 0,33% |
| ac protocatechuique | | 0,06% | 0,43% | 0,05% |
| ac benzoique | | 0,37% | 1,48% | 0,48% |
| Acides cinnamiques | | | | |
| ac. p-coumarique | | 0,31% | 2,48% | 1,40% |
| ac caffeic | | 0,07% | 0,49% | 0,06% |
| acid ferulic | | 0,05% | 0,20% | 0,07% |
| Anthocyanes | Eq cyanidol | 1,03% | nd | 0,25% |
| | Eq | | | |
| Flavonols | quercetine | 3,24% | nd | 1,70% |
| Proanthocyanidols | | | | |
| | DMAC | 16,10% | nd | 5,70% |
| | Pharmacopée | | | |
| | Européene | 88,6% | nd | 35,50% |

Parmi les fractions obtenues les fractions Ib et II peuvent être assemblées avec le concentré liquide Ic contenant l'essentiel des acides organiques de la canneberge permettant ainsi de formuler des extraits secs spécifiques contenant à la fois des polyphénols et des acides polaires.

### Exemple 3. Formulations comprenant l'extrait selon l'invention

Différent types de comprimés contenant les extraits développés sont envisageables, le but général de cette démarche étant à la fois, d'augmenter le potentiel bactéricide et bactériostatique des différents composants actifs de la canneberge directement sous forme d'acides phénoliques et de procyanidols agglomérants au niveau du colon soit sous forme d'acides phénoliques une fois absorbés dans la circulation générale et excrétés dans l'urine.

Il est important de rappeler ici qu'au niveau du colon, la conservation du pouvoir tannant des proanthocyanidols de type B et A, implique que les fonctions ortho-diphénoliques qu'ils comportent n'aient pas été oxydées au préalable et/ou mobilisées par les protéines et acides aminés du bolus alimentaire.

Typiquement pour limiter ces interactions au cours du transit plusieurs solutions peuvent être envisagées :
- limiter l'apport protéique, au moment de la cure et au cours de la prise des actifs de canneberge, pourra ainsi être favorable à l'efficacité des extraits de canneberge développés.
- en favorisant des formes galéniques à libération entérale prolongée.
- compléter la formule du complément alimentaire par un réducteur capable de ralentir l'oxydation des polyphénols typiquement l'acide ascorbique peut jouer ce rôle. En outre son pouvoir réducteur peut contribuer directement ou indirectement à la réduction colonique de l'acide quinique en acide benzoïque.

Les compositions de comprimés classique peuvent être développées en favorisant une libération entérale prolongée afin de limiter les interactions En conséquence les compositions galéniques retenues seront importantes pour cibler la flore uropathogénique sensible à l'effet des composés phénoliques de canneberge, à la fois :
- Les acides phénoliques directement présents dans l'extrait employé,
- L'acide quinique précurseur d'acide benzoïque produit par réduction par la flore intestinale,
- Les flavonols, et anthocyanes, ainsi que les flavanols monomères et proanthocyanidols oligomères comportant des liaisons type B et de type A fermentescibles par la flore intestinale, précurseurs d'acides phénoliques.
- L'effet bactériostatique, agglomérant des tannins proanthocyanidols de canneberge peu fermentescibles polymères c'est-à-dire les proanthocyanidols de plus haut poids moléculaires comportant préférentiellement plusieurs liaisons de type A.

Le comprimé est avalé, il se dissout et doit donc être absorbé dans tout le tractus gastro-intestinal. Une grande variété de supports peuvent être utilisés dont le lactose, le phosphate de calcium, l'amidon, la cellulose microcristalline, des celluloses modifiées comme par exemple l'hydroxypropyl méthylcellulose et l'hydroxyéthylcellulose. Des formules avec des agents d'enrobage tels que sucre, vernis ou cire pour masquer le goût peuvent aussi être envisagées afin d'augmenter la résistance du comprimé lors du passage de l'estomac. Ces revêtements rendant le comprimés résistants aux acides de l'estomac et tels qu'ils se désintègrent que dans le duodénum, le jéjunum et dans le côlon à la suite de l'action des enzymes et/ou du pH alcalin.

Les comprimés cibles utilisant les extraits de canneberge développés une préparation comprimé qui contiennent généralement:
- 5 à 25 % de l'extrait de canneberge (substance active);
- 0 à 10% d'agent réducteur typiquement de l'acide acide ascorbique.
- 70 à 85% de charges matricielle permettant la cohésion et la compression, typiquement des polymères à base cellulosique,
- 0 à 15% des composés qui assurent la désintégration facile, la désagrégation et la dissolution du comprimé à partir l'estomac ou l'intestin.
- 0 à 10% d'agents lubrifiants, des agents de glissement, des cires et agents d'enrobages, et de liants complémentaires.
- Le temps de désagrégation peut être modifié pour obtenir un effet rapide ou pour la libération prolongée.

Comme décrit ci-après les différentes fractions, en synergie avec la pharmacocinétique de libération entérale des comprimés permettent de maitriser l'utilisation de l'ensemble des actifs de la canneberge.

**Tableau 3 : Exemples de composition de comprimés de 500 mg à libération entérale prolongée spécialement formulés dans le cadre de traitement curatif, d'un traitement synergique complet, d'un traitement préventif, et permettant un transfert métabolique permanent des actifs de canneberge.**

| Dans le cadre d'une cure de 15 jours (2 x 500 mg / jour) | | Traitement curatif | | Traitement Synergique complet | | Traitement préventif | |
|---|---|---|---|---|---|---|---|
| Vitesse de dissolution | | Dissolution rapide | | Dissolution moyenne | | Dissolution lente | |
| Mélange | Type | Précurseurs métabolisables | | Précurseurs métabolisables et tannins | | Essentiellement tannins Peu métabolisé | |
| | | *mg* | % | *mg* | % | *mg* | % |
| Extrait de canneberge | Fraction I | 100 | 20% | 60 | 12% | 20 | 4% |
| Extrait de canneberge | Fraction II | 20 | 4% | 60 | 12% | 100 | 20% |
| Acide ascorbique | | 40 | 8% | 40 | 8% | 40 | 8% |
| Hydroxypropylyméthycellulose | Metoloses 90SH 4000SR | 50 | 10% | 70 | 14% | 90 | 18% |
| Diluant | Microcel | 2175 | 44% | 217,5 | 44% | 217,5 | 44% |
| | Lactose | 55 | 11% | 35 | 7% | 15 | 3% |
| Talc | | 10 | 2% | 10 | 2% | 10 | 2% |
| Silice colloïdale | Aerosil | 2,5 | 0,50% | 2,5 | 0,50% | 2,5 | 0,50% |
| Stéarate de Mg | | 5 | 1% | 5 | 1% | 5 | 1% |
| Total | | 500 | 100% | 500 | 100% | 500 | 100% |

Comme décrit dans le **Tableau 3** précédent, les voies d'extraction et les différentes sources de polyphénols de canneberge (fruit, jus, marc exposées précédemment permettent de différencier plusieurs compositions comportant les activités bactériostatiques et bactéricides d'intérêt dans le cadre :
- d'un traitement de type cure rapide plus particulièrement bactéricide,
- d'un traitement synergique bactériostatique et bactéricide,
- d'un traitement « anti-récidive » ou dit de « prévention » essentiellement bactériostatique.

Il est connu que le risque d'apparition d'infections urinaire est favorisé par une faible consommation d'eau. Favoriser une consommation d'eau plus élevée dans le cadre d'un traitement (au moins 2L par jour) avec les extraits de l'invention permet de formuler soit des solutions oro-dispersibles ou des boissons diluées particulièrement bien adaptées au traitement envisagé. Les extraits simples et/ou leurs mélanges synergiques décrits précédemment sont d'abord mélangés avec des fibres *ie.* des colloïdes résistants à l'hydrolyse digestive supérieure (pH acide stomacal, enzymes de l'intestin grêle), permettant de privilégier la libération des tannins de canneberge au niveau du colon.

**Tableau 4 : exemple de composition de boisson diluée et sa forme de reconstitution dite « boisson instantanée »**

| Composition de boisson instantanée | | boisson à reconstituer dilué en g/L |
|---|---|---|
| | % en g/100g | |
| Extrait de canneberge | 1,6% | 0,25 |
| Amidon résistant prégélifié | 15,6% | 2,5 |
| Maltodextine | 6,2% | 1 |
| Gomme de xanthane | 0,9% | 0,15 |
| acide citrique | 12,5% | 2 |
| saccharose | 62,3% | 10 |
| acesulpham K ; sucralose | 0,05% | 0,01 |
| Chlorure de sodium | 0,6% | 0,1 |
| Total | 100,0% | 16,05 |

### Exemple 4. Effets antimicrobiens de l'extrait selon l'invention

Les effets antimicrobiens de l'extrait selon l'invention ont été testés, et comparés à ceux d'extraits plus simples, comprenant :
- **Fraction Ib** telle qu'obtenue à l'Exemple 1 et caractérisée à l'Exemple 2 : fraction comprenant principalement des Proanthocyanidols (PACs) d'un degré de polymérisation moyen d'environ 4, ainsi que des anthocyanes. Cette fraction ne contient que très peu d'acides organiques (voir **Tableau 2** ci-dessus). L'effet antibactérien de cette fraction peut être lié aux PACs et/ou aux anthocyanes.
- **Fractions Ic et II (Ic+II)** telles qu'obtenues à l'Exemple 1 et caractérisées à l'Exemple 2 : mélange comprenant une petite proportion de PACs d'un degré de polymérisation moyen d'environ 8, ainsi qu'une forte proportion d'acides organiques (voir **Tableau 2** ci-dessus). L'effet antibactérien de ce mélange est principalement lié aux acides organiques présents (et notamment aux acides phénoliques les plus apolaires), et éventuellement partiellement aux PACs.

En particulier, la capacité des fractions Ib, (Ic+II), été d'un mélange 50/50 en poids des fractions Ib et (**I**c+**II**) à inhiber la prolifération de différents microorganismes a été mesurée afin de mettre en évidence une éventuelle synergie entre les deux types d'extraits, l'un étant plutôt axé sur les acides organiques, l'autre plutôt sur les PACs et les anthocyanes.

Cinq microorganismes ont été choisis :
- Trois espèces de bactéries connues pour être impliquées dans les infections du tractus urinaire :
   ∘ *Escherichia coli* (ATCC 8739) : cette bactérie gram-négative est responsable d'une proportion importante des infections du tractus urinaire (Orenstein et al., 1999, Shigemura et al., 2005) et il est donc essentiel que l'extrait selon l'invention ait une action inhibitrice sur cette bactérie.
   ∘ *Pseudomonas aeruginosa* (ATCC 9027) : cette bactérie gram-négative est responsable d'une proportion plus ou moins importante (selon l'origine de l'infection et l'origine géographique du patient) des infections du tractus urinaire (Orenstein et al., 1999, Shigemura et al., 2005), en particulier des infections compliquées. En effet, elle conduit souvent à des infections persistantes, chroniques, résistantes aux antibiotiques et finalement récidivantes. Il serait donc également utile que l'extrait selon l'invention ait une action inhibitrice sur cette bactérie.
   ∘ *Staphylococcus aureus* (ATCC 6538) : cette bactérie gram-positive est également retrouvée dans certaines infections du du tractus urinaire (Shigemura et al., 2005). De plus, certaines souches sont résistantes aux antibiotiques classiques et il serait donc également utile que l'extrait selon l'invention ait une action inhibitrice sur cette bactérie.
- Deux souches de champignon :
   ∘ *Candida albicans* (ATCC 10231) : ce champignon est responsable d'un certain nombre d'infections du tractus urinaire, en particulier chez les patients à risques du fait de leur âge avancé, de maladies chroniques comme le diabète, ou de traitements prolongés par des traitements immunosuppresseurs ou anticancéreux (Krcmery et al., 1999).
   ∘ *Aspergillus brasiliensis* (ATCC 16404) : ce champignon n'est pas particulièrement connu pour être associé à des infections du tractus urinaire, mais constitue un modèle d'étude pour quantifier des effets sur les moisissures.

Les souches choisies correspondent en outre à des souches recommandées dans les méthodes réglementaires actuelles pour les tests de l'efficacité de conservation, et de contamination des aliments ou de produit d'hygiène.

Les microorganismes ont été inoculés dans un milieu de culture adéquat et cultivées en plaques 96 puits. Un comptage des microorganismes a été effectué à jours 1, 3, 7, 14 et 21 avec un dosage de triphényltétrazolium (TTC) pour les trois souches bactériennes et pour *Candida albicans*. Ce test repose sur la transformation du TTC blanc en triphenylformazan (TPF) rouge par les cellules vivantes en multiplication. La détection du TPF permet alors d'estimer le nombre de microorganismes présents dans le milieu. Pour *Aspergillus brasiliensis,* un comptage sur boîte conventionnel est effectué.

Le comptage des microorganismes est réalisé soit directement sur la culture, soit après filtration de la culture pour éliminer les agrégats. En effet, les PACs sont connus pour agréger les bactéries en formant des amas en culture, limitant ainsi la formation de biofilms ou l'interaction des bactéries avec les muqueuses infectées. L'identification de la présence de tels agrégats est donc importante, et les résultats obtenus après filtration les plus représentatifs des effets globaux des extraits testés.

Les résultats obtenus sont présentés sur les **Figures 3** à **6** pour quatre des microorganismes testés.

### Escherichia coli

Les **Figures 3A** et **3B** montrent respectivement la croissance des bactéries dans un milieu de culture en absence d'extrait, en présence de la fraction Ib à 5 g/L, de la fraction (Ic+II) à 5 g/L, ou du mélange de la fraction Ib et de la fraction (**I**c+**II**) à 5g/L total (soit 2,5 g/L de fraction Ib et 2,5 g/L de fraction (Ic+II)). Les résultats correspondant à la moyenne des résultats obtenus avec chacune des fractions **I**b, d'une part, et (**I**c+**II**) d'autre part, sont également indiqués. Les résultats sont présentés pour une mesure sans filtration **(****Figure 3A****)** ou avec filtration des aggrégats **(****Figure 3B****).**

En absence de filtration, la fraction Ib est plus efficace que la fraction (**I**c+**II**) pour inhiber la croissance d'*E*. *Coli* in vitro. L'extrait combiné (mélange des fractions Ib et (Ic+II)) est presque aussi efficace que la fraction Ib seule, alors même qu'il ne contient que moitié de la concentration en fraction Ib par rapport à la fraction Ib seule. D'ailleurs, les résultats obtenus avec l'extrait combiné sont bien meilleurs que la moyenne des résultats obtenus avec chacune des fractions Ib et (**I**c+**II**) séparément. Ces résultats suggèrent un effet de synergie.

Lorsque le comptage est réalisé après filtration des agrégats, la fraction Ib est là encore plus.efficace que la fraction (**I**c+**II**) pour inhiber la croissance d'*E*. *Coli* in vitro. L'extrait combiné (mélange des fractions Ib et (Ic+II)) est aussi efficace que la fraction Ib seule, alors même qu'il ne contient que moitié de la concentration en fraction Ib par rapport à la fraction Ib seule. De plus, les résultats obtenus avec l'extrait combiné sont bien meilleurs que la moyenne des résultats obtenus avec chacune des fractions Ib et (**I**c+**II**) séparément. Ces résultats suggèrent là encore un effet de synergie entre les deux fractions testées (Ib/Ic+II).

### Pseudomonas aeruginosa

La **Figure 4A** montre la croissance des bactéries dans un milieu de culture en absence d'extrait, en présence de la fraction Ib à 0,5 g/L, de la fraction (Ic+II) à 0,5 g/L, ou du mélange de la fraction Ib et de la fraction (**I**c+**II**) à 0,5g/L total (soit 0,25 g/L de fraction Ib et 0,25 g/L de fraction (Ic+II)), le comptage étant réalisé sans filtration des agrégats susceptibles d'être présents dans la culture.

La fraction Ib est légèrement plus efficace que la fraction (**I**c+**II**) pour inhiber la croissance de *Pseudomonas aeruginosa* in vitro. L'extrait combiné (mélange des fractions Ib et (Ic+II)) est aussi efficace que la fraction Ib seule, alors même qu'il ne contient que moitié de la concentration en fraction Ib par rapport à la fraction Ib seule. En outre, les résultats obtenus avec l'extrait combiné sont meilleurs que la moyenne des résultats obtenus avec chacune des fractions Ib et (**I**c+**II**) séparément. Ces résultats suggèrent un effet de synergie.

La **Figure 4B** montre la croissance des bactéries dans un milieu de culture en absence d'extrait, en présence de la fraction Ib à 0,1 g/L, de la fraction (**I**c+**II**) à 0,1 g/L, ou du mélange de la fraction Ib et de la fraction (**I**c+**II**) à 0,1g/L total (soit 0,05 g/L de fraction Ib et 0,05 g/L de fraction (Ic+II)), le comptage étant réalisé après filtration des agrégats susceptibles d'être présents dans la culture.

La fraction(Ic+II) est cette fois plus efficace que la fraction Ib pour inhiber la croissance de *Pseudomonas aeruginosa* in vitro. L'extrait combiné (mélange des fractions Ib et (Ic+II)) est plus efficace que chacune des fractions Ib ou (**I**c+**II**) seule, alors même qu'il ne contient que moitié de la concentration en fraction Ib par rapport à la fraction Ib seule, puisqu'il agit plus rapidement que chacune des fractions Ib ou (**I**c+**II**) seule. Ces résultats montrent clairement l'existence d'un effet de synergie entre les deux fractions testées (Ib/Ic+II).

### Staphylococcus aureus

Les **Figures 5A** et **5B** montrent respectivement la croissance des bactéries dans un milieu de culture en absence d'extrait, en présence de la fraction Ib à 5 g/L, de la fraction (**I**c+**II**) à 5 g/L, ou du mélange de la fraction Ib et de la fraction (**I**c+**II**) à 5g/L total (soit 2,5 g/L de fraction Ib et 2,5 g/L de fraction (Ic+II)). Les résultats correspondant à la moyenne des résultats obtenus avec chacune des fractions **I**b, d'une part, et (**I**c+**II**) d'autre part, sont également indiqués. Les résultats sont présentés pour une mesure sans filtration **(****Figure 5A****)** ou avec filtration des agrégats **(****Figure 5B****).**

Dans les deux cas, chacun des extraits séparément et l'extrait combinés sont extrêmement efficaces pour inhiber la croissance de *Staphylococcus aureus* in vitro, à tel point que les conditions testées ne permettent pas de mettre en évidence une synergie entre les deux extraits, chacun des extraits étant déjà efficace au maximum. On ne peut cependant pas exclure la présence d'une synergie, qui pourrait peut-être être démontrée en utilisant des concentrations plus faibles d'extraits. Les effets des combinaisons des fractions phénoliques sont clairement souche dépendant et *Staphylococcus aureus,* souche Gram positive, comporte une paroi particulièrement sensible aux effets des fractions testés ici.

### Candida albicans

Les **Figures 6A** et **6B** montrent respectivement la croissance des champignons dans un milieu de culture en absence d'extrait, en présence de la fraction Ib à 0,5 g/L, de la fraction (**I**c+**II**) à 0,5 g/L, ou du mélange de la fraction Ib et de la fraction (**I**c+**II**) à 0,5g/L total (soit 0,25 g/L de fraction Ib et 0,25 g/L de fraction (Ic+II)). Les résultats correspondant à la moyenne des résultats obtenus avec chacune des fractions **I**b, d'une part, et (**I**c+**II**) d'autre part, sont également indiqués. Les résultats sont présentés pour une mesure sans filtration **(****Figure 6A****)** ou avec filtration des agrégats **(****Figure 6B****).**

En absence de filtration, la fraction Ib est nettement plus efficace que la fraction (**I**c+**II**) pour inhiber la croissance de *Candida albicans* in vitro. L'extrait combiné (mélange des fractions Ib et (Ic+II)) possède une activité inhibitrice intermédiaire, cependant meilleure que la moyenne des résultats obtenus avec chacune des fractions Ib et (**I**c+**II**) séparément.

Lorsque le comptage est réalisé après filtration des agrégats, la fraction Ib est là encore plus efficace que la fraction (**I**c+**II**) pour inhiber la croissance de *Candida albicans* in vitro. L'extrait combiné (mélange des fractions Ib et (Ic+II)) est plus efficace que chacune des fractions Ib ou (**I**c+**II**) seule, alors même qu'il ne contient que moitié de la concentration en fraction Ib par rapport à la fraction Ib seule, puisqu'il agit plus rapidement que chacune des fractions Ib ou (Ic+II) seule. Ces résultats montrent clairement l'existence d'un effet de synergie entre les deux fractions testées (Ib/Ic+II).

### Aspergillus brasiliensis

Il n'a pas été observé d'inhibition significative pour aucun des extraits testés.

### Conclusion

Les résultats présentés ci-dessus montrent clairement que l'extrait selon l'invention, qui combine les fractions **I**b, Ic, et **II**, possède une activité antimicrobienne sur trois bactéries et un champignon connus pour être responsables d'infections du tractus urinaire.

De plus, au moins dans certaines conditions, un effet de synergie entre la fraction Ib riche en PACs d'un degré de polymérisation moyen d'environ 4 et comprenant également des anthocyanes, d'une part, et la fraction Ic+II riche en acides organiques et comprenant également une proportion de PACs d'un degré de polymérisation moyen d'environ 8, d'autre part, a pu être mise en évidence, démontrant ainsi tout l'intérêt de l'extrait selon l'invention, qui combine les différents produits actifs de la canneberge sous forme concentrée, pour améliorer le traitement ou la prévention des infections du tractus urinaire.

Il convient de noter que la synergie entre les différents composants actifs de l'extrait selon l'invention pourrait être encore plus prononcée in vivo.

En effet, in vivo, l'effet des acides organiques, et notamment des acides phénoliques les plus apolaires comme acide benzoïque, est potentiellement limité dans les urines du fait de leur conjugaison avec de la glycine pour former de l'acide hippurique lorsqu'ils sont dans la circulation entéro-hépatique, avant leur excrétion dans les urines.

Or, l'extrait selon l'invention comprend, outre des acides phénoliques, différents précurseurs de ces acides, et notamment de l'acide benzoïque. C'est le cas de l'acide quinique, qui peut être transformé en acide benzoïque par aromatisation réductrice. C'est également le cas des oligomères de PACs, qui ne passent pas la barrière intestinale, mais sont fermentés par la flore intestinale et clivés pour former des acides phénoliques à chaines courtes. Ainsi, outre leur effet d'agrégation des microorganismes dans le tractus digestif, les PACs sont également utiles en tant que précurseurs d'acides phénoliques. De plus le degré de polymérisation des PACS a une influence importante sur leur fermentescibilité par la flore intestinale, comme cela a pu être démontré pour les PAC de type B de la pomme (Bazzocco et al., 2008). Les PACs oligomères sont plus enclins à produire des acides phénoliques, alors que les plus hauts polymères moins fermentescibles peuvent eux inhiber la flore par agrégation. Cet effet est aussi responsable de la réduction des populations de flores dans les expérimentations après filtration comparativement à la réduction sans filtration. Dans le cas de *Pseudomonas aeruginosa* sans filtration à 0,5 g/L, l'effet de la fraction (Ic+II) seule est plus faible que la fraction Ib seule **(****Figure 4A****).** En comparaison, l'effet pour les mêmes fractions à 0,1 g/L après filtration est inversé. Après filtration la fraction (**I**c+**II**) seule est plus efficace, alors qu'elle comporte moins de PAC que la fraction Ib mais plus polymérisés, formant ainsi efficacement des agrégats. Ces effets sont particulièrement adaptés pour la formulation de cures, de traitements d'attaques ou de traitement de fond antirécidive en modifiant les proportions des différentes frations telles que décrites dans le **Tableau 3.**

Enfin c'est aussi le cas des anthocyanes et/ou anthocyanidols, qui sont comme les oligomères de PACs fermentés par la flore intestinale et clivés pour former des acides phénoliques à chaines courtes dans le colon, dont des acides benzoïques.

La combinaison dans l'extrait selon l'invention de la présence d'acides phénoliques et de divers précurseurs conduisant plus ou moins rapidement à de nouveaux acides phénoliques, a pour avantage de conduire à une plus forte concentration d'acides phénolique, soutenue dans le temps, permettant ainsi potentiellement de saturer le système de conjugaison des acides phénoliques avec de la glycine pour former de l'acide hippurique, des acides phénoliques non conjugués, plus actifs, pouvant ainsi atteindre les urines, ce qui a peu de chances de se produire lorsque seuls des acides phénoliques sont présents.

### RÉFÉRENCES BIBLIOGRAPHIQUES

Appeldoorn, M. M., Vincken, J. P., Gruppen, H., and Hollman, P. C. (2009) Procyanidin dimers A1, A2, and B2 are absorbed without conjugation or methylation from the smalt intestine of rats, J Nutr 139, 1469-1473;
Blumenthal M, Hall T, Goldberg A, Kunz T, Kinda K, editors. 2003. The ABC Clinical Guide to Herbs. Austin (TX): American Botanical Council.
Cong, D., Fong, A. K., Lee, R., and Pang, K. S. (2001) Absorption of benzoic acid in segmental régions of the vascularly perfused rat small intestine préparation, Drug Metab Dispos 29, 1539-1547;
EP0752871B1;
EP1014969B1;
EP2033641A1 ;
EP2108268A1;
Bazzocco, S.; Mattila, I., Guyot, S.; Renard, C.M.G.C.; Aura, A-M. Factors affecting the conversion of apple polyphenols to phenolic acids and fruit matrix to short-chain fatty acids by human faecal microbiota in vitro. European Journal of Nutrition vol. 47 issue 8 December 2008. p. 442 - 452.
Gu, L., Kelm, M., Hammerstone, J. F., Beecher, G., Cunningham, D., Vannozzi, S., and Prior, R. L. (2002) Fractionation of polymeric procyanidins from lowbush blueberry and quantification of procyanidins in selected foods with an optimized normal-phase HPLC-MS fluorescent detection method, J Agric Food Chem 50, 4852-4860;
Keppler K, Humpf HU. Metabolism of anthocyanins and their phenolic degradation products by the intestinal microflora. Bioorg Med Chem. 2005 Sep 1;13(17):5195-205.
Krcmery S, Dubrava M, Krcmery V Jr. Fungal urinary tract infections in patients at risk. Int J Antimicrob Agents. 1999 May;11(3-4):289-91.
Orenstein R, Wong ES. Urinary tract infections in adults. Am Fam Physician. 1999 Mar 1;59(5):1225-34, 1237.
Ou, K., Percival, S. S., Zou, T., Khoo, C., and Gu, L. (2012) Transport of cranberry A-type procyanidin dimers, trimers, and tetramers across monolayers of human intestinal epithelial Caco-2 cells, J Agric Food Chem 60, 1390-1396;
Pratt, D.E., Powers, J.J. and Somaatmadja, D. (1960) Anthocyanins. I. The influence of strawberry and grape anthocyanins on the growth of certain bacteria. Food Research 25, 26±32.
Sanchez-Patan, F., Bartolome, B., Martin-Alvarez, P. J., Anderson, M., Howell, A., and Monagas, M. (2012) Comprehensive assessment of the quality of commercial cranberry products. Phenolic characterization and in vitro bioactivity, J Agric Food Chem 60, 3396-3408,
Shigemura K, Tanaka K, Okada H, Nakano Y, Kinoshita S, Gotoh A, Arakawa S, Fujisawa M. Pathogen occurrence and antimicrobial susceptibility of urinary tract infection cases during a 20-year period (1983-2002) at a single institution in Japan. Jpn J Infect Dis. 2005 Oct;58(5):303-8.
US2010/028468;
WO2010/121203 ;
WO96/30033

## Revendications

1. Extrait de canneberge, avantageusement désestérifié, comprenant :
• 5 à 20% en poids, avantageusement 10 à 15% en poids, de proanthocyanidols (PACs) par rapport au poids total de l'extrait sec (mesurés par la méthode de BL-DMAC),
• 2 à 12% en poids, avantageusement 3 à 10% en poids, avantageusement strictement plus de 5% et moins de 10% en poids d'acides organiques par rapport au poids total de l'extrait sec, dont :
∘ 1-10% en poids, avantageusement 1-5% en poids, avantageusement 1 - 3% en poids d'acide quinique par rapport au poids total de l'extrait sec,
∘ 0,5-8% en poids, avantageusement 1-4% en poids, d'acides phénoliques par rapport au poids total de l'extrait sec,
• au moins 0,5% en poids, avantageusement au moins 1% en poids' d'anthocyanes et/ou d'anthocyanidols par rapport au poids total de l'extrait sec,
• 1-10% en poids de sucres par rapport au poids total de l'extrait sec,
• 1-10% en poids, avantageusement 2-5% en poids, de flavonols, sous forme glycosylée /ou aglycone.

2. Extrait selon la revendication 1, **caractérisé en ce que** les polymères de PACs possédant un degré de polymérisation supérieur ou égal à 4 représentent au moins 30% en poids, par rapport au poids de tous les PACs présents dans l'extrait.

3. Procédé de préparation d'un extrait selon l'une quelconque des revendications 1-2, comprenant les étapes suivantes :
a) Enrichissement d'une fraction hydrosoluble de canneberge en composés polyphénoliques apolaires, d'une part, et en acides organiques, d'autre part, pour obtenir une fraction I,
b) Enrichissement d'une fraction non-hydrosoluble de canneberge en polymères de PACs, pour obtenir une fraction **II**,
c) Assemblage des fractions I et II obtenues aux étapes a) et b) respectivement, dans des proportions correspondant à un rapport en poids (fraction **I**/fraction **II**) compris entre 30/70 et 70/30, et
d) Digestion enzymatique par au moins une estérase.

4. Procédé selon la revendication 3, **caractérisé en ce que** :
• la fraction hydrosoluble utilisée à l'étape a) est un jus de canneberge, éventuellement concentré, et la fraction non-hydrosoluble utilisée à l'étape b) est un marc de canneberge, ou
• La fraction hydrosoluble utilisée à l'étape a) et la fraction non-hydrosoluble utilisée à l'étape b) ont été obtenues à partir de baies de canneberge entières, par :
i) Broyage de baies de canneberge entières, et
ii) Décantation et séparation des fractions hydrosoluble et non-hydrosoluble.

5. Procédé selon la revendication 3 ou la revendication 4, **caractérisé en ce que** la fraction I est obtenue à l'étape a) par les étapes suivantes :
a1) séparation entre les composés apolaires et les composés polaires par :
• adsorption de la fraction hydrosoluble sur un support apolaire,
• lavage avec un solvant polaire et collecte de la fraction polaire Ia, et
• élution et collecte de la fraction apolaire Ib ;
a2) séparation entre les acides organiques et les sucres présents dans la fraction polaire Ia par :
• adsorption de la fraction Ia sur une résine échangeuse d'ions,
• lavage avec un solvant aqueux,
• élution avec un solvant acide et collecte de la fraction Ic comprenant les acides organiques ;
a3) assemblage des fractions Ib et Ic pour former la fraction I.

6. Procédé selon l'une quelconque des revendications 3-5, **caractérisé en ce que** la fraction II est obtenue à l'étape b) par un traitement comprenant une étape d'extraction des polymères de PACs de la fraction non-hydrosoluble par un solvant organique, de préférence suivie d'étapes de clarification, distillation et concentration de la solution enrichie ainsi obtenue.

7. Procédé selon l'une quelconque des revendications 3-6, **caractérisé en ce que** l'assemblage de la fraction I obtenue à l'étape a) et de la fraction II obtenue à l'étape b) est réalisé par simple mélange des fractions I et II, sans élimination d'une partie de l'une des fractions.

8. Procédé selon l'une quelconque des revendications 3-7, **caractérisé en ce que** l'étape d) de digestion enzymatique par au moins une estérase est réalisée :
• avant l'étape a), soit au cours de la préparation des fractions hydrosoluble et non-hydrosoluble sur le broyat, de préférence liquéfié, soit sur la fraction hydrosoluble et la fraction non-hydrosoluble, de préférence liquéfiées,
• entre les étapes a) et b) et l'étape c) d'assemblage final, sur les fractions I et II, ou
• à la fin du procédé, sur l'extrait assemblé obtenu à l'issue de l'étape c).

9. Extrait susceptible d'être obtenu par le procédé selon l'une quelconque des revendications 3-8.

10. Extrait selon l'une quelconque des revendications 1-2 et 9, qui est un extrait sec, et qui se présente avantageusement sous forme de poudre, de comprimé, de gélule, de granules, ou de concentré liquide.

11. Composition alimentaire ou nutraceutique, comprenant un extrait selon l'une quelconque des revendications 1-2 et 9-10.

12. Composition pharmaceutique, comprenant un extrait selon l'une quelconque des revendications 1-2 et 9-10.

13. Extrait selon l'une quelconque des revendications 1-2 et 9-10, pour son utilisation en tant que médicament.

14. Extrait selon l'une quelconque des revendications 1-2 et 9-10, pour son utilisation dans la prévention ou le traitement des infections urinaires, en particulier par les bactéries uropathogéniques, les bactéries gram positives, et d'autres bactéries choisies parmi *Proteus mirabilis,* les espèces du genre *Staphylococcus,* les espèces du genre *Saprophyticus,* et les espèces du genre *Klebsiella.*

15. Extrait selon l'une quelconque des revendications 1-2 et 9-10, pour son utilisation dans la prévention des récidives d'infections urinaires.

## Patentansprüche

1. Cranberry-Extrakt, vorzugsweise entestert, umfassend:
• 5 bis 20 Gew.-%, vorzugsweise 10 bis 15 Gew.-%, Proanthocyanidole (PACs) in Bezug zum Gesamtgewicht des Trockenextrakts (gemessen mit der BL-DMAC-Methode),
• 2 bis 12 Gew.-%, vorzugsweise 3 bis 10 Gew.-%, vorzugsweise strikt über 5 % und weniger als 10 Gew.-% organische Säuren in Bezug zum Gesamtgewicht des Trockenextrakts, davon:
∘ 1-10 Gew.-%, vorzugsweise 1-5 Gew.-%, vorzugsweise 1-3 Gew.-% Chinasäure in Bezug zum Gesamtgewicht des Trockenextrakts,
∘ 0,5-8 Gew.-%, vorzugsweise 1-4 Gew.-%, Phenolsäuren in Bezug zum Gesamtgewicht des Trockenextrakts,
• mindestens 0,5 Gew.-%, vorzugsweise mindestens 1 Gew.-% Anthocyane und/oder Anthocyanidole in Bezug zum Gesamtgewicht des Trockenextrakts,
• 1-10 Gew.-% Zucker in Bezug zum Gesamtgewicht des Trockenextrakts,
• 1-10 Gew.-%, vorzugsweise 2-5 Gew.-%, Flavonole in glycosylierter und/oder Aglykon-Form.

2. Extrakt nach Anspruch 1, **dadurch gekennzeichnet, dass** die PACs-Polymere, die einen Polymerisationsgrad von über oder gleich 4 besitzen, mindestens 30 Gew.-% in Bezug zum Gewicht aller im Extrakt vorhandenen PACs darstellen.

3. Herstellungsverfahren eines Extrakts nach einem der Ansprüche 1-2, umfassend die folgenden Schritte:
a) Anreichern einer wasserlöslichen Cranberry-Fraktion mit apolaren Polyphenolverbindungen einerseits und mit organischen Säuren andererseits, um eine Fraktion I zu erhalten,
b) Anreichern einer nicht wasserlöslichen Cranberry-Fraktion mit PACs-Polymeren, um eine Fraktion II zu erhalten,
c) Kombinieren der Fraktionen I und II, erhalten in den Schritten a) und b), jeweils in Anteilen, die einem Gewichtsverhältnis (Fraktion I/Fraktion II) zwischen 30/70 und 70/30 inklusive entsprechen, und
d) enzymatische Verdauung mittels mindestens einer Esterase.

4. Verfahren nach Anspruch 3, **dadurch gekennzeichnet, dass**:
• die in Schritt a) verwendete wasserlösliche Fraktion ein eventuell konzentrierter Cranberry-Saft ist und die nicht wasserlösliche Fraktion von Schritt b) ein Cranberry-Mark ist, oder
• die in Schritt a) verwendete wasserlösliche Fraktion und die nicht wasserlösliche Fraktion von Schritt b) aus ganzen Cranberrys erhalten wurden mittels:
i) Zerkleinern der ganzen Cranberry-Beeren, und
ii) Dekantieren und Trennen der wasserlöslichen und nicht wasserlöslichen Fraktionen.

5. Verfahren nach Anspruch 3 oder Anspruch 4, **dadurch gekennzeichnet, dass** die Fraktion I in Schritt a) mittels der folgenden Schritte erhalten wird:
a1) Trennen zwischen den apolaren Verbindungen und den polaren Verbindungen mittels:
• Adsorption der wasserlöslichen Fraktion auf einem aoplaren Träger,
• Waschen mit einem polaren Lösungsmittel und Sammeln der polaren Fraktion la, und
• Elution und Sammeln der apolaren Fraktion Ib,
a2) Trennen zwischen den organischen Säuren und den Zuckern, die in der polaren Fraktion Ia vorhanden sind, mittels:
• Adsorption der Fraktion la auf einem Ionentauscherharz,
• Waschen mit einem wässrigen Lösungsmittel,
• Elution mit einem sauren Lösungsmittel und Sammeln der Fraktion Ic, die die organischen Säuren umfasst,
a3) Kombinieren der Fraktionen Ib und Ic, um die Fraktion I zu bilden.

6. Verfahren nach einem der Ansprüche 3-5, **dadurch gekennzeichnet, dass** die Fraktion II in Schritt b) mittels einer Verarbeitung erhalten wird, die einen Extraktionsschritt der PACs-Polymere der nicht wasserlöslichen Fraktion mittels eines organischen Lösungsmittels umfasst, vorzugsweise gefolgt von einem Schritt der Reinigung, Destillation und Konzentration der derart erhaltenen angereicherten Lösung.

7. Verfahren nach einem der Ansprüche 3-6, **dadurch gekennzeichnet, dass** die Kombination der Fraktion I aus Schritt a) und der Fraktion II aus Schritt b) durch einfaches Mischen der Fraktionen I und II ohne Entfernung eines Teils einer der Fraktionen erfolgt.

8. Verfahren nach einem der Ansprüche 3-7, **dadurch gekennzeichnet, dass** der Schritt d) der enzymatischen Verdauung mittels mindestens einer Esterase durchgeführt wird:
• vor Schritt a), entweder während der Herstellung der wasserlöslichen und nicht wasserlöslichen Fraktionen auf dem vorzugsweise verflüssigten zerkleinerten Material oder auf der vorzugsweise verflüssigten wasserlöslichen Fraktion und nicht wasserlöslichen Fraktion,
• zwischen den Schritten a) und b) und dem Schritt c) der abschließenden Kombination auf den Fraktionen I und II, oder
• am Ende des Verfahrens auf dem kombinierten Extrakt nach Beendigung von Schritt c).

9. Extrakt, das mittels des Verfahrens nach einem der Ansprüche 3-8 gewinnbar ist.

10. Extrakt nach einem der Ansprüche 1-2 und 9, der ein Trockenextrakt ist und der in vorteilhafter Weise in Form von Pulver, Tablette, Kapsel, Granulat oder Flüssigkonzentrat vorliegt.

11. Nahrungs- oder nutrazeutische Zusammensetzung, umfassend einen Extrakt nach einem der Ansprüche 1-2 und 9-10.

12. Pharmazeutische Zusammensetzung, umfassend einen Extrakt nach einem der Ansprüche 1-2 und 9-10.

13. Extrakt nach einem der Ansprüche 1-2 und 9-10 für seine Verwendung als Arzneimittel.

14. Extrakt nach einem der Ansprüche 1-2 und 9-10 für seine Verwendung bei der Vorbeugung oder Behandlung von Harnwegsinfektionen, insbesondere durch uropathogene Bakterien, grampositive Bakterien und andere Bakterien, ausgewählt aus *Proteus mirabilis,* den Arten der Gattung *Staphylococcus,* den Arten der Gattung *Saprophyticus* und den Arten der Gattung *Klebsiella.*

15. Extrakt nach einem der Ansprüche 1-2 und 9-10 für seine Verwendung bei der Vorbeugung von Rezidiven von Harnwegsinfektionen.

## Claims

1. Cranberry extract, advantageously deesterified, comprising:
• 5 to 20% by weight, advantageously 10 to 15% by weight, of proanthocyanidins (PACs) in relation to the total weight of the dry extract (measured by the BL-DMAC method),
• 2 to 12% by weight, advantageously 3 to 10% by weight, advantageously strictly more than 5% and less than 10% by weight of organic acids in relation to the total weight of the dry extract, including:
∘ 1-10% by weight, advantageously 1-5% by weight, advantageously 1-3% by weight of quinic acid in relation to the total weight of the dry extract,
∘ 0.5-8% by weight, advantageously 1-4% by weight, of phenolic acids in relation to the total weight of the dry extract,
• at least 0.5% by weight, advantageously at least 1% by weight of anthocyans and/or anthocyanidins in relation to the total weight of the dry extract,
• 1-10% by weight of sugars in relation to the total weight of the dry extract,
• 1-10% by weight, advantageously 2-5% by weight, of flavonols, in glycosylated and/or aglycone form.

2. Extract according to claim 1, **characterized in that** the polymers of PACs having a degree of polymerization greater than or equal to 4 represent at least 30% by weight, in relation to the weight of all PACs present in the extract.

3. Process for preparing an extract according to any one of claims 1-2, comprising the following steps:
a) Enriching a water-soluble fraction of cranberry in non-polar polyphenolic compounds, on the one hand, and in organic acids, on the other hand, to obtain fraction I,
b) Enriching a water-insoluble fraction of cranberry in polymers of PACs, to obtain fraction II,
c) Combining fractions I and II obtained in steps a) and b), respectively, in proportions corresponding to a weight ratio (fraction I/fraction II) ranging between 30/70 and 70/30, and
d) Enzymatic digestion with at least one esterase.

4. Process according to claim 3, **characterized in that**:
• the water-soluble fraction used in step a) is cranberry juice, optionally concentrated, and the water-insoluble fraction used in step b) is cranberry marc, or
• The water-soluble fraction used in step a) and the water-insoluble fraction used in step b) were obtained from whole cranberries, by:
i) Crushing whole cranberries, and
ii) Decanting and separating the water-soluble and water-insoluble fractions.

5. Process according to claim 3 or claim 4, **characterized in that** fraction I is obtained in step a) by the following steps:
a1) separating non-polar compounds and polar compounds by:
• adsorption of the water-soluble fraction on a non-polar support,
• washing with a polar solvent and collecting polar fraction Ia, and
• eluting and collecting non-polar fraction Ib;
a2) separating organic acids and sugars present in polar fraction Ia by:
• adsorption of fraction Ia on ion-exchange resin,
• washing with aqueous solvent,
• eluting with acidic solvent and collecting fraction Ic comprising organic acids;
a3) Combining fractions Ib and Ic to form fraction I.

6. Process according to any one of claims 3-5, **characterized in that** fraction II is obtained in step b) by treatment comprising a step of extracting polymers of PACs from the water-insoluble fraction with organic solvent, preferably followed by steps of clarification, distillation and concentration of the enriched solution thus obtained.

7. Process according to any one of claims 3-6, **characterized in that** the combining of fraction I obtained in step a) and fraction II obtained in step b) is carried out by simple mixing of fractions I and II, without removing part of one of the fractions.

8. Process according to any one of claims 3-7, **characterized in that** step d) of enzymatic digestion with at least one esterase is carried out:
• before step a), either during preparation of the water-soluble and water-insoluble fractions on crushed material, preferably liquefied, or on the water-soluble fraction and the water-insoluble fraction, preferably liquefied,
• between steps a) and b) and final combination step c), on fractions I and II, or
• at the end of the process, on the combined extract obtained at the conclusion of step c).

9. Extract obtainable by the process according to any one of claims 3-8.

10. Extract according to any one of claims 1-2 and 9, which is a dry extract and which is advantageously provided in powder, tablet, capsule, granules or liquid concentrate form.

11. Food or nutraceutical composition, comprising an extract according to any one of claims 1-2 and 9-10.

12. Pharmaceutical composition, comprising an extract according to any one of claims 1-2 and 9-10.

13. Extract according to any one of claims 1-2 and 9-10, for use as a drug.

14. Extract according to any one of claims 1-2 and 9-10, for use in the prevention or treatment of urinary tract infections, in particular by uropathogenic bacteria, Gram-positive bacteria, and other bacteria selected from *Proteus mirabilis,* species of the genus *Staphylococcus,* species of the genus *Saprophyticus,* and species of the genus *Klebsiella.*

15. Extract according to any one of claims 1-2 and 9-10, for use in the prevention of the recurrence of urinary tract infections.
